# EUROPÄISCHE PATENTSCHRIFT

(11) **EP 2 258 283 B1**
(45) Veröffentlichungstag und Bekanntmachung des Hinweises auf die Patenterteilung: **28.08.2013**
(21) Anmeldenummer: 09162034.4
(22) Anmeldetag: 05.06.2009
(51) Int. Cl.: A61B 17/16, A61B 17/17, A61F 2/36, A61B 19/00

(54) **Werkzeugaufsatz für medizintechnische Anwendungen**
Tool attachment for medical applications
Attachement d'outils pour applications médicales

(43) Veröffentlichungstag der Anmeldung: 08.12.2010
(73) Patentinhaber: BrainLAB AG, 85622 Feldkirchen (DE)
(72) Erfinder: Tuma, Gregor, 81677, München (DE)
(74) Vertreter: Schwabe - Sandmair - Marx

(56) Entgegenhaltungen:
- WO-A-2006/100458
- US-A1- 2005 234 332
- US-A1- 2005 234 465
- US-A1- 2008 009 697

## Beschreibung

Die vorliegende Erfindung bezieht sich auf einen Werkzeugaufsatz für medizintechnische Anwendungen, insbesondere zum Bearbeiten von Knochenteilen.

Jüngere Entwicklungen in der Chirurgie bzw. Orthopädie haben zur Konstruktion von Hüftkappenprothesen geführt, bei deren Einsatz in den Körper eines Patienten lediglich die Oberfläche bzw. äußere Schicht des Hüftgelenkkopfes bzw. des Oberschenkelkopfes (caput femoris) entfernt und durch eine Metallkappe ersetzt wird. Dieses Verfahren zum Entfernen der Knochenoberfläche (engl.: Resurfacing) unterscheidet sich von den herkömmlichen Verfahren dadurch, dass bislang der Oberschenkelkopf einfach abgesägt und vollständig durch eine Schaftprothese ersetzt wurde. Das Resurfacing wurde bislang händisch erledigt, d.h. das Gerät zum Entfernen, insbesondere Abfräsen der Knochenoberfläche wurde händisch von einem Operateur geführt, wobei sich große Unsicherheiten aufgrund einer Bewegung der Fräsbohrmaschine relativ zum Oberschenkel ergaben.

Bei dem bisher üblichen Fräsen der Knochenoberfläche trat das so genannte "Femoral Notching" auf: Durch die händische Führung des Fräskopfes kann in den Schenkelhals versehentlich eine Kerbe eingefräst werden, die bei postoperativer Belastung des Hüftgelenkes zu einem Schenkelhalsbruch führen kann.

US 2008/009697 A1 offenbart ein handgeführtes chirurgisches Instrument 500, das eine Ausrichtungshilfe 115 und ein Schneidwerkzeug 102 aufweist. Das chirurgische Instrument kann gemäß Absatz [0142] drahtlos übertragene Steuersignale empfangen.

US 2005/0234465 A1 offenbart eine handgeführte Säge zur Bearbeitung eines Femurkopfes, die fest an einem Schablonenblock angebracht werden kann, so dass die Säge entlang der durch die Schablone vorgegebenen Richtungen geführt werden kann.

Aufgabe der Erfindung ist es eine Verbesserung bei der Bearbeitung einer Knochenoberfläche mit einem Werkzeugaufsatz zu ermöglichen. Diese Aufgabe wird von der Lehre der unabhängigen Patentansprüche gelöst, wobei die abhängigen Ansprüche sich auf bestimmte Ausführungsformen dieser Lehre beziehen. Die Merkmale unterschiedlicher Ausführungsformen können untereinander kombiniert werden.

Die Erfindung stellt einen Fräskopf bereit, der an eine im Schenkelhals angebrachte Fräskopfhalterung, insbesondere eine Fräskopfführung angekoppelt werden kann. Die im Schenkelhals angebrachte Fräskopfhalterung (beispielsweise ein Befestigungsschaft) kann insbesondere eine Referenzsternhalterung umfassen, so dass der daran angekoppelte Fräskopf entsprechend einer Planung für die Operation eine hinreichend genaue Position besitzt, um den Knochen an der gewünschten Stelle zu bearbeiten. Ein automatisches, insbesondere einem Steuerprogramm folgendes (also kontrolliertes) Führen des Fräskopfes wird ermöglicht.

Die vorliegende Erfindung bezieht sich auf einen Werkzeugaufsatz für medizintechnische Anwendungen, insbesondere zum Fräsen und/oder Bohren und/oder Schneiden von Knochenteilen. Unter Fräsen wird im Rahmen dieser Erfindung ein zerspanendes bzw. spanabhebendes Verfahren zur Bearbeitung von Oberflächen, insbesondere Knochenoberflächen, verstanden. Bohren soll ein Verfahren bezeichnen, bei dem eine Vertiefung und/oder ein Kanal und/oder ein Loch in eine körperhafte Struktur, insbesondere einen Knochen, eingearbeitet wird. Unter dem Schneiden von Knochenteilen wird ein mechanisches Trennverfahren verstanden, mit dem ein Knochenteil in mehrere, wenigstens aber zwei Knochenteile aufgeteilt wird. Der erfindungsgemäße Werkzeugaufsatz beinhaltet wenigstens einen Bearbeitungsabschnitt zum Bearbeiten, also Fräsen und/oder Bohren und/oder Schneiden, eines Knochenteils. Ein Fräsen kann insbesondere so erfolgen, dass ein Oberschenkelkopf entlang einer Oberfläche gefräst wird, die rotationssymmetrisch um die Längsachse des Oberschenkelkopfes gelegen ist. Der Oberschenkelkopf wird hierin beispielhaft für jegliche Körperstrukturen, insbesondere Knochenteile (z.B. Condylen, Schulterblatt, etc.) genannt. Der Bearbeitungsabschnitt beinhaltet dazu beispielsweise eine Fräse, wie sie für die chirurgische Knochenbearbeitung gängig ist. Diese Fräse kann insbesondere so ausgelegt sein, dass sie die Oberfläche des Schenkelhalses entlang einer rotationssymmetrischen Oberfläche fräst. Die rotationssymmetrische Oberfläche kann eine Oberfläche einer rotationssymmetrischen geometrischen Figur (geometrischen Grundform) darstellen, die rotationssymmetrisch bezüglich einer Längsache des Oberschenkelkopfes ist. Hier kommen beispielsweise ein Zylindermantel und/oder eine Kegeloberfläche (ein Kegelmantel) in Frage. Erfindungsgemäß kann auch eine Kombination aus einem Zylindermantel und einem Kegelmantel erstellt werden: Beispielsweise kann das proximale Ende des Oberschenkelkopfes spitz zulaufend, also kegelförmig gefräst werden, wobei sich an die Kegelbasis distal eine Zylinderform des verbleibenden Oberschenkelkopfes und/oder Schenkelhalses in der bearbeiteten Form anschließt. Das erfindungsgemäße Fräsen mittels eines Werkzeugaufsatzes, der in einem chirurgischen Navigationsverfahren (engl.: Image Guided Surgery - IGS) von einem Roboter geführt wird, ermöglicht eine genaue Oberflächenbearbeitung. Auf diese Weise entsteht eine rotationssymmetrische Form des Oberschenkelhalses, insbesondere eine der oben genannten Grundformen. Der Bearbeitungsabschnitt bzw. die Fräse kann also so ausgelegt sein, dass sie auf einer Oberfläche der Grundform, deren Längsachse auf der Längsachse des Schenkelhalses und/oder parallel zu der Längsachse des Schenkelhalses liegt, rotiert. Das heißt, der Bearbeitungsabschnitt fräst eine Oberfläche des Schenkelhalses, die zum Ende der Knochenbearbeitung auf einer Oberfläche, insbesondere einer Mantelfläche, einer solchen Grundform liegt. Eine solche Mantelfläche ist also um die Längsachse des Oberschenkelkopfes und/oder um eine Achse rotationssymmetrisch, die parallel zu der Längsachse des Oberschenkelkopfes liegt. Unter der Längsachse des Oberschenkelkopfes ist insbesondere eine Achse zu verstehen, entlang der der Oberschenkelkopf Rotationssymmetrie bzw. annähernde Rotationssymmetrie aufweist. Weiter beinhaltet der Werkzeugaufsatz eine Befestigungsmöglichkeit zum Befestigen des Werkzeugaufsatzes an einer Steuereinrichtung, die den Werkzeugaufsatz führt (lenkt) und/oder antreibt. Die Steuereinrichtung steuert also insbesondere die Lage des Werkzeugaufsatzes im Raum. Diese Befestigungsmöglichkeit sorgt beispielsweise durch eine formschlüssige und/oder kraftschlüssige und/oder stoffschlüssige mechanische Verbindung zu der Steuereinrichtung dafür, dass der Werkzeugaufsatz kontrolliert geführt werden kann. Die Steuereinrichtung kann beispielsweise den Arm eines Roboters umfassen, wobei der Roboter manuell von einem Anwender und/oder über ein Computerprogramm gesteuert werden kann. Die Steuereinrichtung kann auch einen Handgriff zum manuellen Führen des Werkzeugaufsatzes beinhalten, damit der Werkzeugaufsatz beim Hin- und/oder Wegführen an eine Bearbeitungsposition des Werkzugaufsatzes, in der der Oberschenkelkopf bearbeitet werden soll, durch einen Bearbeiter manuell geführt werden kann. Die Fein- oder Grobsteuerung der Lage des Werkzeugaufsatzes, d.h. die Steuerung einer Lage des Werkzeugaufsatzes in der näheren Umgebung des Oberschenkelkopfes bzw. zu bearbeitenden Körperteils, kann dann mit Hilfe der Steuereinrichtung durchgeführt werden. Die Steuereinrichtung kann einen Motor (z.B. einen Elektromotor) und/oder ein Getriebe zum Antreiben des Werkzeugaufsatzes umfassen. So ist es zum Beispiel möglich, einen Roboter derart anzusteuern, dass er den Werkzeugaufsatz an Positionen bewegt, die durch ein chirurgisches Navigationsverfahren ermittelt werden. Dazu ist an der Steuereinrichtung und/oder dem Werkzeugaufsatz eine Markervorrichtung angebracht. Die Ermittlung der Positionen kann derart erfolgen, dass eine Oberfläche des Oberschenkelkopfes vor der Bearbeitung mit einem Zeigegerät (Pointer) abgegriffen wird (registriert wird). Das Abgreifen von patientenspezifischen Punkten/Landmarken auf der Oberfläche eines realen Knochens mit einem Pointer wird Registrieren genannt. Die Steuerung des Werkzeugaufsatzes relativ zu der zu bearbeitenden Oberfläche erfolgt dann über einen Vergleich der ermittelten Position der Steuereinrichtung und/oder des Werkzeugaufsatzes sowie der (insbesondere registrierten) Lage (also Position und Orientierung im Raum) der zu bearbeitenden Oberfläche.

Ein Pointer ist ein Stab mit einem oder mehreren, vorteilhaft zwei daran befestigten Marker, wobei der Pointer dazu verwendet werden kann, z.B. im Rahmen eines Registrierverfahrens einzelne Koordinaten, insbesondere Raumkoordinaten (also dreidimensionale Koordinaten) an einem Körperteil, abzugreifen. Dabei führt ein Benutzer den Pointer (insbesondere einen Teil des Pointers, der eine definierte - vorteilhaft feste - Lage bezüglich des wenigstens einen an dem Pointer angebrachten Markers innehat) so an die den Koordinaten entsprechende Position, dass über eine Erfassung des Markers an dem Pointer durch ein chirurgisches Navigationssystem die Position des Pointers bestimmt werden kann. Insbesondere ist die relative Lage zwischen den Markern des Pointers und dem zum Abgreifen von Koordinaten verwendeten Teil des Pointers (inbesondere der Pointerspitze) bekannt. Das chirurgische Navigationssystem ermöglicht dann die Zuordnung der Position (der dreidimensionalen Koordinaten) zu einer vorbestimmten Körperstruktur, wobei die Zuordnung automatisch und/oder durch Eingreifen des Benutzers erfolgen kann.

Somit sind die Position und Form des Oberschenkelkopfes vor der Bearbeitung mit dem erfindungsgemäßen Werkzeugaufsatz vorzugsweise bekannt, und entsprechende Daten werden an eine Datenverarbeitungsvorrichtung gesendet und beispielsweise dort gespeichert. Die Datenverarbeitungsvorrichtung besteht dabei aus einem Computer, d.h. insbesondere einem Chip bzw. Prozessor, einer Speichereinheit und gegebenenfalls einer Anzeigevorrichtung (wie einem Monitor und/oder einem akustischen Sender wie beispielsweise einem Lautsprecher). Im Rahmen der Erfindung wird für die Navigation während einer Operation eine weitere Markervorrichtung, die eine oder mehrere einzelne Markervorrichtungen und/oder einen Referenzstern umfassen kann, an einem mit dem Oberschenkelkopf ortsfest verbundenen Instrument, insbesondere der Fräskopfführung, angebracht.

Eine Markervorrichtung kann ein Referenzstern, ein Pointer und/oder ein einzelner oder mehrere Marker sein.

Aufgabe eines Markers ist, von einer Markererfassungseinrichtung (z.B. einer Kamera oder einem Ultraschallempfänger) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Ein Referenzstern bezeichnet eine Einrichtung, an der mehrere, vorteilhaft drei Marker angebracht sind. Die Marker sind dabei ortsfest und vorteilhaft lösbar an dem Referenzstern angebracht, so dass eine bekannte (vorteilhaft feste) relative Lage der Marker zueinander entsteht. Die relative Lage der Marker zueinander kann für jeden im Rahmen eines chirurgischen Navigationsverfahrens verwendeten Referenzstern individuell unterschiedlich sein, um anhand der relativen Lage der Marker zueinander eine Identifikation des dazugehörigen Referenzsterns durch ein chirurgisches Navigationssystem zu ermöglichen. Damit können dann auch die Gegenstände (z. B. Instrumente und/oder Körperteile) identifiziert bzw. voneinander unterschieden werden, an welche der Referenzstern angebracht ist. Der Referenzstern dient dazu, in einem chirurgischen Navigationsverfahren eine Mehrzahl von Markern an einem Gegenstand (beispielsweise einem Knochen oder einem medizinischen Instrument) anzubringen, um die Lage des Gegenstandes im Raum (d.h. seine Position und/oder Ausrichtung) erfassen zu können. Ein solcher Referenzstern umfasst insbesondere eine Anbringmöglichkeit an dem Gegenstand (beispielsweise eine Schelle und/oder ein Gewinde) und /oder ein Halteelement, das für einen Abstand der Marker von dem Gegenstand sorgt (um insbesondere die Sichtbarkeit der Marker für eine Markererfassungscinrichtung zu unterstützen) und/oder mit dem Halteelement mechanisch verbundene Markerhalter, an die die Marker angebracht werden können. Wo es aus dem Zusammenhang klar wird, kann der Begriff Referenzstern auch einen Referenzstern mit wenigstens einem daran angebrachten Marker bezeichnen. Ein solches System aus einem Referenzstern und wenigstens einem Marker kann auch Markerstern genannt werden.

Unter einem Markerhalter versteht man eine Anbringmöglichkeit für einen einzelnen Marker, die dazu dient den Marker an ein Instrument, ein Körperteil und/oder ein Halteelement eines Referenzstern anzubringen. Das Anbringen kann dabei ortsfest und vorteilhaft lösbar sein. Die Form eines Markerhalters kann beispielsweise stäbchenförmig bzw. zylinderförmig sein. An dem dem Marker zugewandten Ende des Markerhalters kann eine Befestigungsmöglichkeit für die Markervorrichtung vorgesehen sein (wie etwa ein Einrastmechanismus), der eine kraftschlüssigen und/oder formschlüssigen Sitz der Markervorrichtung an dem Markerhalter unterstützt.

Eine erste Markervorrichtung (im Folgenden auch "Knochenmarkervorrichtung" genannt) wird in einer relativ zum Oberschenkelkopf festen Position angebracht. Dies kann durch direkte mechanische Befestigung der Knochenmarkervorrichtung am Knochen oder durch Befestigung der Knochenmarkervorrichtung an einem fest mit dem Knochen mechanisch verbundenen Anschlussstück geschehen. Die in diesem Rahmen auftretenden mechanischen Verbindungen sind vorzugsweise lösbar. Das Anschlussstück unterstützt vorzugsweise einen ortsfesten Sitz der Knochenmarkervorrichtung relativ zum zu bearbeitenden Knochenteil. Vorzugsweise ist an der Steuereinrichtung, insbesondere einem ersten Roboterarm ortsfest eine zweite Markervorrichtung angebracht. Eine solche Markervorrichtung wird hierin auch als Robotermarkervorrichtung bezeichnet. Der erste Roboterarm kann mithilfe der Robotermarkervorrichtung so relativ zum Oberschenkelkopf navigiert bzw. bewegt werden, dass es möglich ist eine Werkzeugaufsatzführung, insbesondere eine Fräskopfführung, die an dem ersten Roboterarm geführt wird und dessen Position relativ zu der Robotermarkervorrichtung vorbekannt und vorzugsweise fest ist, entlang der Längsachse des Oberschenkelkopfes bzw. Schenkelhalses in den Knochen einzuführen. Alternativ oder zusätzlich zu der Robotermarkervorrichtung kann eine Markervorrichtung an der Fräskopfführung angebracht sein; diese alternative oder zusätzliche Markervorrichtung wird dann als Führungsmarkervorrichtung oder Fräskopfführungsmarkervorrichtung bezeichnet. Im Folgenden wird eine Fräskopfführung beispielhaft für eine Werkzeugaufsatzführung, die an einem Knochenteil befestigt werden kann, angeführt. Erfindungsgemäß möglich ist aber auch die Verwendung von anderen Werkzeugaufsatzführungen, insbesondere von Führungen für Bohr- und/oder Schneidaufsätze.

Die Fräskopfführung erlaubt ein (lösbares) mechanisches Verbinden (insbesondere Ankoppeln) des Werkzeugaufsatzes mit der Fräskopfführung, so dass sich vermittelt über die Fräskopfführung eine (insbesondere ortsfeste und/oder lösbare) feste mechanische Verbindung des Werkzeugaufsatzes mit dem Oberschenkelkopf ergibt. Dadurch wird während der Bearbeitung eine relativ zum Oberschenkelkopf bzw. der zu bearbeitenden Oberfläche feste Position des Werkzeugaufsatzes unterstützt und insbesondere gewährleistet, insbesondere selbst wenn sich die zu bearbeitende Oberfläche im Raum bewegt. Die Fräskopfführung ist dabei vorzugsweise so gestaltet, dass im Zustand der Verbindung mit dem Werkzeugaufsatz die zur Bearbeitung des Oberschenkelkopfes notwendigen Bewegungen (z. B. Rotation) des wenigstens einen Bearbeitungsabschnitts nicht verhindert werden.

Die Führungsmarkervorrichtung ist während des Bewegungs- und/oder Einführvorgangs vorzugsweise an der Fräskopfführung mechanisch ortsfest und/oder lösbar befestigt. Somit ist es möglich, eine Markervorrichtung für die gemeinsame Navigation der Fräskopfführung und des Roboterarms zu nutzen, wenn die Fräskopfführung eine feste relative Position zum Roboterarm hat. Dies kann auch der Fall sein, wenn die Fräskopfführung lösbar aber ortsfest mit dem Roboterarm mechanisch (z. B. über eine Steckverbindung und/oder Schraubverbindung, also formschlüssig und/oder kraftschlüssig) verbunden ist. Die Führungsmarkervorrichtung kann somit, falls erwünscht, die Robotermarkervorrichtung zu dem vorgenannten Zweck ersetzen.

Nach dem Einführen der Fräskopfführung in den Oberschenkelkopf, so dass dieser eine ortsfeste Lage relativ zum Oberschenkelkopf einnimmt, kann ein zweiter Roboterarm (der mit dem ersten Roboterarm identisch sein kann der insbesondere die Robotermarkervorrichtung umfasst), an dem der erfindungsgemäße Werkzeugaufsatz aufgesetzt ist, so relativ zur Fräskopfführung bzw. der Führungsmarkervorrichtung insbesondere durch Detektion der Robotermarkervorrichtung und der Führungsmarkervorrichtung navigiert werden, dass der Bearbeitungsabschnitt in einer zum rotationssymmetrischen, insbesondere zylindrischen Fräsen des Oberschenkelkopfes geeigneten Position an den Knochen angelegt wird bzw. werden kann. Der Roboterarm kann bewegt werden, indem er mit einer Bewegungseinheit verbunden wird. Diese Bewegungseinheit kann z.B. ein Motor, insbesondere ein Elektromotor sein, der Steuersignale von einem Rechner, insbesondere dem Rechner des chirurgischen Navigationssystems, erhält und diese über eine Bewegungsmechanik an den Roboterarm weitergibt.

Vorzugsweise ist beim Anlegen des Bearbeitungsabschnitts an den Knochen die Führungsmarkervorrichtung noch an der Fräskopfführung befestigt. Damit der Werkzeugaufsatz die oben beschriebene mechanische Verbindung mit der Fräskopfführung eingehen kann, kann an dem Werkzeugaufsatz eine Aussparung zum Einführen einer Markervorrichtung vorgesehen sein. Die Markervorrichtung ist vorteilhaft die Führungsmarkervorrichtung, die vorzugsweise ortsfest relativ zur Fräskopfführung an der Fräskopfführung angebracht ist. Die Aussparung unterstützt insbesondere einen lösbar ortsfesten Sitz des Werkzeugaufsatzes relativ zur Führungsmarkervorrichtung. Die Aussparung kann als eine Vertiefung in einer Oberfläche des Werkzeugaufsatzes eingeformt sein. Sie kann aber auch als Kanal durch den Werkzeugaufsatz geformt sein, um das Einführen der Führungsmarkervorrichtung zu ermöglichen. Vorteilhaft hat die Aussparung die Form eines Schlitzes in dem Werkzeugaufsatz, der sich parallel zu einer Längsachse bzw. Rotationsachse (insbesondere Zylinderachse) des Werkzeugaufsatzes erstreckt. Die Aussparung und die Führungsmarkervorrichtung (insbesondere ein Halteelement der Führungsmarkervorrichtung) sind vorteilhaft zumindest teilweise kongruent zueinander ausgebildet. Dadurch wird ein fester mechanischer, insbesondere formschlüssiger Sitz des Werkzeugaufsatzes an der Führungsmarkervorrichtung unterstützt. Der Sitz ist vorteilhaft lösbar. Eine Ausbildung der Aussparung, die zumindest teilweise kongruent zur Fräskopfführung ist, ist erfindungsgemäß ebenfalls möglich. Dadurch wird ein fester mechanischer, insbesondere formschlüssiger Sitz des Werkzeugaufsatzes mittels der Aussparung an der Fräskopfführung unterstützt, wenn auch ein Bestandteil des Fräskopfes insbesondere zusätzlich zu einem Bestandteil der Führungsmarkervorrichtung in die Aussparung eingelegt wird. Dadurch ist ein spielfreier Sitz des Werkzeugaufsatzes an der Führungsmarkervorrichtung bzw. an der Fräskopfführung möglich, insbesondere wird eine drehfeste und vorteilhaft lösbare Verbindung zwischen dem Werkzeugaufsatz und der Führungsmarkervorrichtung bzw. der Fräskopfführung hergestellt bzw. unterstützt. Der Schlitz ist vorteilhaft dort offen, wo er in Richtung des Oberschenkelkopfes weist, so dass eine am Oberschenkelkopf angeordnete Knochenmarkervorrichtung oder eine Führungsmarkervorrichtung in dem Werkzeugaufsatz bewegt bzw. beim Anlegevorgang in den Werkzeugaufsatz eingeführt werden kann. Die Aussparung kann in einen Stützabschnitt und/oder einen Bearbeitungsabschnitt des Werkzeugaufsatzes geformt sein.

Der Stützabschnitt des Werkzeugaufsatzes bezeichnet hierbei einen ruhenden Teil, insbesondere einen Teil des Werkzeugaufsatzes, an dem der Bearbeitungsabschnitt tragend befestigt ist. Der Stützabschnitt umfasst insbesondere einen Verbindungsabschnitt. Insbesondere ruht der Stützabschnitt während der Bearbeitung des Knochenteils relativ zum Knochenteil, verhält sich also ortsfest relativ zum Knochenteil. Der Verbindungsabschnitt dient dem ortsfesten und vorteilhaft lösbaren mechanischen Verbinden des Werkzeugaufsatzes mit der Fräskopfführung. Der Verbindungsabschnitt kann beispielsweise eine Einformung in den Stützabschnitt umfassen, die vorteilhaft zumindest teilweise kongruent zur Form der Fräskopfführung ausgebildet ist. Dadurch wird eine feste mechanische, insbesondere formschlüssige Verbindung zwischen Werkzeugaufsatz und Fräskopfführung ermöglicht bzw. unterstützt. Erfindungsgemäß möglich ist auch ein Kupplungsmechanismus, wie z.B. ein Einrastmechanismus, der an dem Verbindungsabschnitt angeordnet ist, und in ein entsprechendes Gegenteil an der Fräskopfführung eingreift bzw. einrastet. Dadurch wird vorteilhaft ein spielfreier Sitz, insbesondere eine drehfeste Verbindung zwischen dem Werkzeugaufsatz und der Fräskopfführung hergestellt bzw. unterstützt.

Vorteilhaft ist die Aussparung nur in den Stützabschnitt eingeformt; dabei ragt der Stützabschnitt vorzugsweise in Richtung des offenen Endes des Werkzeugaufsatzes, das im Betriebszustand in Richtung des Oberschenkelkopfes und insbesondere weg vom Roboterarm weist, über den wenigstens einen Bearbeitungsabschnitt in Längsrichtung des Werkzeugaufsatzes hinaus. Die Aussparung befindet sich vorzugsweise in einer Region des Stützabschnitts, die sich bei der Bearbeitung außerhalb eines Bewegungs- bzw. Bearbeitungsbereichs des wenigstens einen Bearbeitungsabschnitts befindet. Dadurch wird ermöglicht, dass die Führungsmarkervorrichtung, die in die Aussparung hineinragt, während der Bearbeitung in ihrer Position verbleiben kann. Der Werkzeugaufsatz kann dann die Führungsmarkervorrichtung umgreifen und wenigstens der Stützabschnitt eine feste relative Position gegenüber dieser haben, wobei die Führungsmarkervorrichtung sich zugleich außerhalb des Bewegungsbereichs des wenigstens einen Bearbeitungsabschnitts befindet. Damit ist das Vorhandensein der Führungsmarkervorrichtung kein Hindernis für den Betrieb des wenigstens einen Bearbeitungsabschnitts und eine Beschädigung sowohl eines Bearbeitungsabschnittes als auch der Führungsmarkervorrichtung während der Bearbeitung wird so weniger wahrscheinlich. An einem dem Roboterarm zugewandten Ende der Aussparung, insbesondere des Schlitzes kann eine Einformung vorgesehen sein, die einen passgenauen Sitz des Werkzeugaufsatzes an der Führungsmarkervorrichtung unterstützt. Dies kann z.B. eine runde und/oder teilkreisförmige bzw. halbkreisförmige und/oder wenigstens abschnittsweise eckige bzw. polygonale Form insbesondere in Projektion auf eine Ebene sein, wobei die Ebene senkrecht zur Längsrichtung des Haltelements der Führungsmarkervorrichtung liegt, falls letztere ein Referenzstern ist (bzw. senkrecht zur Längsrichtung des Markerhalters im Falle eines einzelnen Markers als Markervorrichtung). Die Einformung kann somit wenigstens teilweise passgenau zu dem Halteelement und/oder dem Referenzstern ausgebildet sein. Es kann ferner ein Einschnappmechanismus (Einrastmechanismus) an der Aussparung vorgesehen sein, der in eine entsprechende Oberflächenform der Führungsmarkervorrichtung bzw. der Fräskopfführung eingreift. Umgekehrt kann auch ein Einrastmechanismus an der Führungsmarkervorrichtung bzw. der Fräskopfführung vorgesehen sein, der an eine Entsprechende Oberflächenform der Aussparung eingreift. Insbesondere können Elemente am dem Roboterarm zugewandten Ende der Aussparung vorgesehen sein, die einen kraftschlüssigen Sitz des Werkzeugaufsatzes an der Führungsmarkervorrichtung unterstützen, z.B. Federn und/oder Klemmen.

Vorteilhaft unterstützt der Verbindungsabschnitt einen ortsfesten Sitz des wenigstens einen Bearbeitungsabschnitts relativ zum Oberschenkelkopf in dem der Verbindungsabschnitt mit der Führungsmarkervorrichtung und oder der Fräskopfführung in Kontakt ist. Gemäß einer Ausführungsform ist der Verbindungsabschnitt zur lösbar festen mechanischen Verbindung mit der Fräskopfführung vorgesehen. Dieser Verbindungsabschnitt kann alternativ zu oder zusätzlich zu der oben genannten, im Bearbeitungsabschnitt vorgesehenen Aussparung vorgesehen sein. Die Aussparung kann auch, wie oben beschrieben im Verbindungsabschnitt vorgesehen sein. Gemäß einer Ausführungsform kann also der Verbindungsabschnitt die Aussparung wenigstens teilweise beinhalten. Dies ist insbesondere vorteilhaft, wenn eine lösbare mechanische Verbindung zwischen dem Werkzeugaufsatz und einem Bestandteil der Führungsmarkervorrichtung hergestellt werden soll. Sind nämlich die Führungsmarkervorrichtung und die Fräskopfführung ausreichend stabil miteinander mechanisch verbunden, kann darauf verzichtet werden, eine direkte lösbare mechanische Verbindung zwischen dem Verbindungsabschnitt und der Fräskopfführung herzustellen, um die Bearbeitung des Knochenteils durchzuführen.

Der Verbindungsabschnitt ist vorteilhaft in bzw. auf einer Längsachse des Werkzeugaufsatzes angeordnet, so dass es im Bearbeitungszustand dem aus dem Knochen herausragenden bzw. offen liegenden Teil der Fräskopfführung gegenüberliegt. Gemäß einer Ausführungsform ermöglicht eine stabile Verbindung des Verbindungsabschnitts einen ortsfesten Sitz des Stützabschnitts, der vorzugsweise den Verbindungsabschnitt oder zumindest mit ihm ortsfest verbunden ist relativ zum zu bearbeitenden Knochenteil, indem der Verbindungsabschnitt vorzugsweise zumindest eine seitliche Verlagerung des Werkzeugaufsatzes relativ zur Bearbeitungsebene und/oder radial zur Längsachse des Werkzeugaufsatzes blockiert. Insbesondere ruht der Verbindungsabschnitt während der Bearbeitung relativ zum zu bearbeitenden Knochenteil. Unter der Längsachse des Werkzeugaufsatzes ist insbesondere eine Achse zu verstehen, entlang der der Werkzeugaufsatz Rotationssymmetrie aufweist und die vorzugsweise parallel zu und/oder auf der Längsachse des Oberschenkelkopfes bei der Bearbeitung des Knochens platziert wird. Gemäß einer anderen Ausführungsform kann eine dem gleichen Zweck dienende Verbindung auch zwischen dem Verbindungsabschnitt und der Führungsmarkervorrichtung hergestellt werden. Der Verbindungsabschnitt kann in, insbesondere an dem Stützabschnitt angeordnet sein. Der Verbindungsabschnitt kann eine Ausnehmung bzw. Vertiefung umfassen bzw. ein Verbindungsteil, das mit der Fräskopfführung in eine insbesondere formschlüssige und/oder kraftschlüssige mechanische Verbindung eintreten kann. Dieses Verbindungsteil kann beispielsweise einen Schnappverschluss bzw. Einschnapp- bzw. Einrastmechanismus umfassen. Dieses Verbindungsteil ist vorzugsweise an einer Stelle des Werkzeugaufsatzes angeordnet, die in der Bearbeitungsposition insbesondere einem Endstück der Fräskopfführung bzw. einem an der Fräskopfführung angeordneten Anschlussstück gegenüberliegt. Dieses Anschlussstück fungiert beispielsweise als Adapter zwischen der Fräskopfführung und dem Werkzeugaufsatz und ragt vorteilhaft aus dem Knochen hervor und befindet sich auf bzw. nahe der Längsachse des Oberschenkelkopfes, verläuft also insbesondere entlang bzw. parallel der Längsachse. Die feste mechanische Verbindung zwischen dem Werkzeugaufsatz und der Fräskopfführung und/oder der Führungsmarkervorrichtung ermöglicht ein formgenaues Bearbeiten bzw. Fräsen der Oberschenkelkopfoberfläche und unterstützt eine präzise Bearbeitung des Schenkelhalses entsprechend eines Operationsplans. Der Verbindungsabschnitt kann z.B. eine runde und/oder kreisförmige und/oder auch polygonale bzw. eckige Vertiefung auf einer dem Oberschenkelkopf zugewandten Oberfläche des Stützabschnitts beinhalten. Diese Vertiefung kann in eine zumindest teilweise passgenaue und/oder formschlüssige und/oder kraftschlüssige Verbindung mit dem gegenüberliegenden (vorteilhaft nicht im Knochen versenkten) Teil der Fräskopfführung treten (z.B. dem Anschlussstück), wozu dieser Teil der Fräskopfführung vorteilhaft eine Form besitzt, die ein Negativ der Vertiefung darstellt. Die Oberflächenform (Topographie) des gegenüberliegenden Teils der Fräskopfführung (insbesondere des Anschlussstücks) ist also so gestaltet, dass sie in die entgegengestzt geformte Oberflächenform (Topographie) des Verbindungsabschnitts bündig bzw. formschlüssig eingreifen kann. Es ist aber auch möglich, eine Vertiefung in den aus dem Knochen hervorstehenden Teil der Fräskopfführung (also in den freistehenden, nicht im Knochen versenkten Teil der Fräskopfführung, insbesondere in das Anschlussstück) einzuformen, die z.B. ringförmig und/oder kreisförmig und/oder auch polygonal bzw. eckig gestaltet ist. Dann ist es zweckmäßig, wenn der Stützabschnitt eine Erhebung aufweist, die beim Anlegen des Werkzeugaufsatzes an die Fräskopfführung in die Vertiefung an dem freistehenden Teil der Fräskopfführung insbesondere passgenau und/oder spielfrei und/oder drehfest eingreifen kann. Wenn der Verbindungsabschnitt eine Vertiefung und/oder Erhebung in runder und/oder kreisförmiger Ausgestaltung aufweist, kann eine Ausbuchtung bzw. Kerbe in der Außenlinie der Vertiefung und/oder Erhebung in ein entsprechendes Negativ auf dem gegenüberliegenden Teil der Fräskopfführung eingreifen, um ein Verdrehen des Werkzeugaufsatzes relativ zur Fräskopfführung und/oder Oberschenkelkopf zu verhindern. Erfindungsgemäß ist aber auch möglich, dass auf der Seite des Werkzeugaufsatzes eine polygonale Erhebung und auf dem freistehenden Teil der Fräskopfführung eine Vertiefung in Form des entsprechenden Negatives eingeformt ist. Dadurch wird ein Verdrillen bzw. Verdrehen des Roboterarms bzw. eines Stützabschnitts relativ zum Oberschenkelkopf bei der Bearbeitung bzw. dem Fräsen erschwert. Zusätzlich zu den Erhebungen und Vertiefungen (aber auch unabhängig vom Vorhandensein dieser) kann an dem Anschlussstück auch ein Schnappverschluss bzw. Einrastmechanismus angeordnet sein, um die Stabilität der Verbindung zwischen Werkzeugaufsatz und Fräskopfführung zu erhöhen. Zusätzlich oder alternativ kann ein Schnappverschluss bzw. Einrastmechanismus an dem Verbindungsabschnitt angeordnet sein, um eine lösbare, feste mechanische Verbindung zwischen diesem und dem Anschlussstück zu unterstützen. Das Anschlussstück hat also insbesondere die Funktion einer Kupplung zwischen dem Werkzeugaufsatz und der Fräskopfführung.

Die Aussparung kann in wenigstens einen Bearbeitungsabsclmitt und gleichzeitig in den Stützabschnitt und/oder den Verbindungsabschnitt eingeformt sein. Die Aussparung läuft z.B. radial von außen nach innen z.B. vom Stützabschnitt über einen Bearbeitungsabschnitt bis zum Verbindungsabschnitt, um wenigstens das Halteelement einer Führungsmarkervorrichtung aufzunehmen. Sie kann aber auch nur in wenigstens einen Bearbeitungsabschnitt oder nur in den Verbindungsabschnitt eingeformt sein. Nach dem Ankoppeln des Werkzeugaufsatzes an die Fräskopfführung und vor der Bearbeitung wird die Führungsmarkervorrichtung dann gemäß einer Ausführungsform entfernt, um nicht ein Hindernis im Bewegungsbereich eines Bearbeitungsabschnitts darzustellen, falls die Führungsmarkervorrichtung im Bewegungsbereich der Bearbeitungsabschnitte und/oder an einem Bearbeitungsabschnitt vorgesehen ist.

Die Aussparung weist vorteilhaft eine Dimensionierung bzw. Breite auf, die insbesondere das Durchführen eines Teils einer Markervorrichtung ermöglicht. Dieser Teil der Markervorrichtung kann z.B. ein Halteelement eines Referenzsterns sein. In diesem Fall ist der Schlitz insbesondere breit genug, dass das Halteelement entlang der Längserstreckung des Schlitzes durch den Schlitz geführt werden kann. Insbesondere bildet beim Hindurchführen des Halteelements durch den Schlitz die Längsrichtung des Halteelements mit der Längsebene des Schlitzes einen rechten Winkel.

Die Lage der Aussparung kann relativ zum Werkzeugaufsatz (insbesondere relativ zum Stützabschnitt) verstellbar sein. Zum Beispiel kann eine schlitzförmige Aussparung in einem relativ zu anderen Komponenten des Werkzeugaufsatzes verschiebbaren Teil des Werkzeugaufsatzes angebracht sein. So kann etwa der Schlitz in einer Platte angeordnet sein, die koplanar zu einer Oberfläche des Stützabschnitts und/oder eines Bearbeitungsabschnitts und/oder des Verbindungsabschnitts in den Stützabschnitt und/oder den Bearbeitungsabschnitt eingefügt ist und innerhalb des Stütz- und/oder Verbindungs- und/oder Bearbeitungsabschnitts verschoben werden kann. Dies ermöglicht die exakte Einstellung der Aussparung auf die Position der in sie einzuführenden Führungsmarkervorrichtung bei gleichzeitiger Unabhängigkeit von z. B. bestimmten Knochenformen. So kann die Aussparung auch an die Markervorrichtung angelegt werden, wenn das zu bearbeitende Knochenteil eine für die Umgreifung durch den Werkzeugaufsatz schwierige Geometrie aufweist.

Es ist erfindungsgemäß möglich, den Stützabschnitt in Teilen auslenkbar gegenüber einer Bearbeitungskonfiguration zu gestalten, so dass insbesondere zwei einander gegenüberliegende Teile eines Stützabschnittes während des Anlegens des Werkzeugaufsatzes an den Oberschenkelkopf um die Markervorrichtung bzw. das Halteelement herum geführt werden können. Die Bearbeitungskonfiguration bezeichnet dabei eine geometrische Einstellung des Werkzeugaufsatzes und/oder seiner Bestandteile, die bei der Bearbeitung des Knochens vorteilhaft ist. Zu unterscheiden von der Bearbeitungskonfiguration ist die Bearbeitungsposition, die die eine Bearbeitung ermöglichende Lage des Werkzeugaufsatzes in Bearbeitungskonfiguration relativ zum zu bearbeitenden Knochenteil beschreibt. So kann ein Teil des Stützabschnitt relativ zu einem anderen Teil des Stützabschnitts verschwenkt, insbesondere von letzterem weggeschwenkt werden, um eine Lücke zu schaffen, in die die Führungsmarkervorrichtung eingeführt werden kann. Nach dem Einführen wenigstens eines Teils der Führungsmarkervorrichtung in die Lücke kann der Stützabschnitt wieder geschlossen werden (d. h. seine zuvor voneinander weg geschwenkten Teile einander angenähert werden), bis er die Führungsmarkervorrichtung wenigstens teilweise umgibt. Diese Konfiguration mit geschlossenen Stützabschnitten ist dann Bestandteil einer Bearbeitungskonfiguration. Die Aussparung kann in diesem Fall zwei halbrunde bzw. halbkreisförmige Ausnehmungen aus dem Stützabschnitt umfassen, wobei jeweils eine der Ausnehmungen auf einem der beiden verschwenkbaren Teile des Stützabschnitts angeordnet ist. Die beiden Ausnehmungen sind dabei insbesondere so angeordnet, dass sie im geschlossenen Zustand des Stützabschnitts einander gegenüberliegen, so dass sie ein Loch bzw. einen Kanal bilden, in dem wenigstens ein Teil der Markervorrichtung liegen kann. Dabei ist es vorteilhaft, wenn die Aussparung (insbesondere der Kanal) zumindest teilweise passgenau und/oder formschlüssig mit der Führungsmarkervorrichtung abschließt, um eine genaue bzw. ortsfeste Positionierung des Werkzeugaufsatzes relativ zur Führungsmarlcervorrichtung zu unterstützen.

Die Aussparung erlaubt vorzugsweise ein exaktes Anlegen des Werkzeugaufsatzes an die Führungsmarkervorrichtung in einer Position, die eine für das Fräsen gewünschte Position darstellt, so dass sich insbesondere bei passgenauem Kontakt zwischen der Führungsmarkervorrichtung und der Aussparung eine vorbestimmte und bekannte relative Lage zwischen der Führungsmarkervorrichtung und dem Werkzeugaufsatz einstellt. Die Ermittlung der Position des Werkzeugaufsatzes (nach dem Anlegen) erfolgt in einem chirurgischen Navigationsverfahren durch einen Vergleich der ermittelten Position des Roboterarms und/oder einer Robotermarkervorrichtung, deren relative Lagen zum Werkzeugaufsatz jeweils bekannt vorzugsweise fest sind, mit der ermittelten Position der Führungsmarkervorrichtung und/oder ein Kombinieren dieses Vergleichs mit der registrierten Form des Oberschenkelkopfes und/oder vor der Operation bekannten geometrischen Daten des Oberschenkelkopfes. Die vorbekannten Daten von wenigstens Teilen des Patientenkörpers können beispielsweise durch bildgebende medizintechnische Verfahren gewonnen werden und werden vorzugsweise dem erfindungsgemäßen Verfahren bereitgestellt. Unter einem solchen Verfahren versteht man vorteilhaft apparative Verfahren der Radiologie, wie etwa Computertomographie (CT), Röntgentomographie, Magnetresonanztomographie (MRT bzw. MRI), herkömmliches Röntgen, Sonographie bzw. Ultrasehalluntersuchungen, Positronen-Emissions-Tomographie. Werden hierin Daten "bereitgestellt", so bedeutet dies, dass sie für die Nutzung durch das erfindungsgemäße Verfahren bereit sind. Diesen Zustand des "bereit seins" können die Daten z.B. durch Erfassen der Daten (z.B. durch Analysegeräte) oder durch Eingeben der Daten (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit sind. Vor dem Bereitstellen, insbesondere vor dem Abspeichern, können die Daten auch in einem Schritt des erfindungsgemäßen Verfahrens bestimmt, insbesondere berechnet werden.

Die zum Bearbeiten bzw. Fräsen geeignete Position des Bearbeitungsabschnitts relativ zum Knochen (Bearbeitungsposition) kann so definiert sein, dass der Bearbeitungsabschnitt den Oberschenkelkopf wenigstens teilweise, insbesondere entlang bzw. parallel zu dessen Längsachse, umfasst. Im Falle eines hohlzylinderförmigen Werkzeugaufsatzes wird der Oberschenkelkopf koaxial wenigstens teilweise von dem Bearbeitungsabschnitt umfasst. Es ist erfindungsgemäß aber auch möglich, dass der Oberschenkelkopf zumindest großteils, insbesondere entlang bzw. parallel zur gesamten Länge seiner Längsachse bzw. von einem Ende seiner Längsachse zu deren anderen Ende, von dem Bearbeitungsabschnitt umfasst wird. Unter Umfassen wird hier verstanden, dass die Knochenoberfläche wenigstens abschnittsweise in wenigstens zwei, vorteilhaft mehreren Richtungen einem Bearbeitungsabschnitt gegenüberliegt. Es liegt also vorzugsweise jedem Bearbeitungsabschnitt ein Abschnitt der Knochenoberfläche im Inneren des hohlzylinderförmigen Werkzeugaufsatzes gegenüber.

Der Werkzeugaufsatz kann auch Zangenform besitzen. Dabei können die Bearbeitungsabschnitte auf wenigstens einem Arm der Zangenform angeordnet sein, der beweglich in dem Werkzeugaufsatz gelagert ist und um den Oberschenkelkopf rotiert, um den Bearbeitungsabschnitt in die Bearbeitungsposition zu verfahren. Der Stützabschnitt kann in diesem Fall in Hohlzylinderform um den wenigstens einen Arm angebracht sein. Der Stützabschnitt kann aber auch flächig und/oder quaderförmig parallel zur Längsachse des Oberschenkelkopfes ausgebildet sein. Der Stützabschnitt kann aber auch in einen Arm eingearbeitet sein bzw. von diesem beinhaltet werden. Der Stützabschnitt kann auch nur einen Teil des Oberschenkelkopfes umfassen, insbesondere ihn im Bearbeitungszustand nicht rotationssymmetrisch umgeben. Es ist vorteilhaft, wenn der Stützabschnitt sich außerhalb des Bewegungsbereichs des wenigstens einen Bearbeitungsabschnitts befindet, so dass die Bewegung des Bearbeitungsabschnitts (insbesondere während der Bearbeitung) nicht durch die Anordnung des Stützabschnitts behindert wird.

Damit der Oberschenkelkopf rotationssymmetrisch, vorteilhaft in Zylinderform bearbeitet werden kann, weist ein beispielsweise hohlzylinderförmiger Werkzeugaufsatz vorteilhaft eine Ausnehmung auf, mit deren Hilfe der Oberschenkelkopf wenigstens teilweise umgriffen werden kann. Vorteilhaft ist der Werkzeugaufsatz hohlzylindrisch ausgebildet, wobei der Zylinder eine offene Grundfläche hat, die zur Bearbeitung dem Oberschenkelkopf zugewandt wird. Somit kann der Werkzeugaufsatz über den Oberschenkelkopf gestülpt werden. Der wenigstens ein Bearbeitungsabschnitt befindet sich dann beispielsweise auf einer inneren Oberfläche bzw. inneren Mantelfläche dieses hohlzylindrischen Werkzeugaufsatzes, um eine Bearbeitung der gegenüberliegenden Knochenoberfläche zu ermöglichen. Der Werkzeugaufsatz besitzt daher beispielsweise die Form eines Hohlzylinders mit einer offenen Grundfläche. Möglich ist aber auch die Gestaltung in Kegelform mit einer offenen Grundfläche, wobei die offene Grundfläche vorteilhaft dem Oberschenkkopf zur Bearbeitung zugewandt wird. In diesem Fall liegen mehrere (wenigstens zwei) Bearbeitungsabschnitte einander nicht parallel gegenüber; vielmehr liegen die Längsachsen der Bearbeitungsabschnitte in einem Winkel zueinander.

Der Werkzeugaufsatz kann ferner einen inneren Stützabschnitt aufweisen, der als Tragabschnitt für den Verbindungsabschnitt wirkt. Der Stützabschnitt kann im Inneren (d. h. in einem vom Bewegungsbereich des wenigstens einen Bearbeitungsabschnitts umgebenen Gebiet) bzw. parallel und/oder entlang einer Längsachse des Werkzeugaufsatzes bzw. Roboterarms angeordnet sein und lineare Gestalt aufweisen. Diese Längsachse wird vorzugsweise in Deckung mit der Längsachse des Oberschenkelkopfes und/oder der Fräskopfführung gebracht, so dass die beiden Längsachsen und/oder in Längsrichtung gelegenen Symmetrieachsen aufeinander und/oder parallel zueinander gelegen sind. Dies erhöht die Stabilität der Anordnung des Werkzeugaufsatzes an dem Oberschenkelkopf bei der Bearbeitung des Knochens. Der lineare Stützabschnitt kann beispielsweise so ausgebildet sein, dass er zylindrische Form bzw. Stabform besitzt und koaxial zu dem Hohlzylinder angeordnet ist, welcher den wenigstens einen Bearbeitungsabschnitt beinhaltet. Der Hohlzylinder bzw. wenigstens eine Bearbeitungsarm mit dem wenigstens einen Bearbeitungsabschnitt kann auf seiner dem Knochen abgewandten Seite wenigstens teilweise von dem Stützabschnitt überdeckt werden. Der Stützabschnitt kann den Verbindungsabschnitt beinhalten. Vorteilhaft erstreckt sich der Stützabschnitt also in Richtung bzw. parallel zu einer Längsachse des Werkzeugaufsatzes und/oder des Roboterarms.

Bestandteil der Erfindung ist auch ein System aus einem oben beschriebenen Werkzeugaufsatz und einer Markervorrichtung, insbesondere einer Führungsmarkervorrichtung. Die Führungsmarkervorrichtung kann dabei mit einer Fräskopfführung, insbesondere mit dem nach Einführen in den Oberschenkelknochen freiliegenden Teil der Fräskopfführung (insbesondere dem Anschlussstück) mechanisch verbunden sein. Dieses System aus einem Werkzeugaufsatz und einer Führungsmarkervorrichtung kann auch ein chirurgisches Navigationssystem umfassen.

Unter einem chirurgischen Navigationssystem wird ein System verstanden, das aus wenigstens einer Markervorrichtung, einem Sender, der elektromagnetische Wellen bzw. Strahlung und/oder Ultraschallwellen emittiert, und einem Empfänger, der elektromagnetische Wellen bzw. Strahlung und/oder Ultraschallwellen empfängt, sowie einer elektronischen Datenverarbeitungsvorrichtung, die an den Empfänger und/oder den Sender angeschlossen ist, besteht. Die Datenverarbeitungsvorrichtung (z. B. ein Computer) umfasst dabei einen Prozessor (CPU), einen Arbeitsspeicher (RAM), vorteilhaft eine Anzeigemöglichkeit (z. B. eine visuelle Anzeigemöglichkeit wie einen Monitor und/oder eine Audioanzeigemöglichkeit wie einen Lautsprecher) sowie vorteilhaft einen permanenten Datenspeicher (z.B. eine Festplatte). Die Datenverarbeitungsvorrichtung verarbeitet dabei Navigationsdaten, die von dem Empfänger an sie weitergegeben werden und kann vorteilhaft Hinweisinformationen über die Anzeigemöglichkeit an einen Anwender ausgeben. Die Navigationsdaten können in dem permanenten Datenspeicher abgelegt werden und beispielsweise mit Daten verglichen werden, die dort zuvor bereitgestellt worden sind.

Bestandteil der Erfindung ist ebenfalls ein medizindatentechnisches Datenverarbeitungsverfahren, insbesondere ein Steuerprogramm zum Positionieren eines oben beschriebenen Werkzeugaufsatzes an einem Knochenteil, insbesondere einem Oberschenkelkopf mittels einer Steuereinrichtung. Das Steuerprogramm läuft dabei vorteilhaft auf einer Datenverarbeitungsvorrichtung, insbesondere einem Computer (beispielsweise einem in einem Prozessor, der einem chirurgischen Navigationssystem zugeordnet ist) ab und kann die folgenden Schritte umfassen: Vorteilhaft werden durch das Steuerprogramm Geometriedaten, insbesondere Daten, die Informationen über die Geometrie des zu bearbeitenden Knochenteils, beispielsweise des Oberschenkelkopfes, vorzugsweise im Rahmen eines Registrierverfahrens mithilfe eines chirurgischen Navigationssystems bereitgestellt, insbesondere eingelesen. Anschließend können diese Geometriedaten in einer Speichereinrichtung des Computers gespeichert werden. Aus den Geometriedaten des Knochenteils wird eine Längsachse des Knochenteils ermittelt. Ferner werden dem Steuerprogramm vorteilhaft Führungsdaten bereitgestellt, die Informationen über die Lage der Führungsmarkervorrichtung bezüglich des Knochenteils, insbesondere bezüglich der Längsachse des Knochenteils umfassen. Dabei wird die Position der Führungsmarkervorrichtung über ein chirurgisches Navigationssystem erfasst. Ferner werden dem Steuerprogramm Lagedaten bereitgestellt, die Informationen über die räumliche Lage des Werkzeugaufsatzes (insbesondere relativ zur Führungsmarkervorrichtung) umfassen. Diese Lagedaten können über eine an dem Werkzeugaufsatz angebrachte Markervorrichtung (also eine Werkzeugmarkervorrichtung) und/oder eine Robotermarkervorrichtung und/oder die bekannten relativen Lagen zwischen Steuereinrichtung und Knochenteil sowie Steuereinrichtung und Werkzeugaufsatz ermittelt werden. Anschließend kann eine Steuereinrichtung, insbesondere ein Roboter, so von dem Steuerprogramm angesteuert werden, dass sein Arbeitsarm eine Markervorrichtung (Führungsmarkervorrichtung) auf bzw. parallel zu der Längsachse positioniert. Weiterhin wird der Werkzeugaufsatz über die Steuereinrichtung an die Führungsmarkervorrichtung herangeführt. Dazu werden dem Steuerprogramm Steuerdaten bereitgestellt, die die Steuereinrichtung veranlassen, den Werkzeugaufsatz and die Führungsmarkervorrichtung heranzuführen. Derartige Steuerdaten beinhalten vorteilhafte Informationen über die Lage des Werkzeugaufsatzes relativ zur Führungsmarkervorrichtung bzw. der Fräskopfführung. Ferner können die Steuerdaten Anweisungen umfassen, die insbesondere darauf hinweisen, wie der Werkzeugaufsatz navigiert bzw. bewegt werden muss, um an die Fräskopfführung angekoppelt werden zu können. Die Bestimmung der Steuerdaten erfolgt vorteilhaft mithilfe einer Datenverarbeitungsvorrichtung, die insbesondere zu dem chirurgischen Navigationssystem gehört, wobei die Datenverarbeitungsvorrichtung die Lagen bzw. Anweisungen vorteilhaft über einen Vergleich der Orte bzw. Positionen von Werkzeugaufsatz und Führungsmarkervorrichtung bzw. Fräskopfführung ermittelt werden. Die Bestimmung der Steuerdaten kann insbesondere auf den Führungsdaten und Lagedaten basieren, wobei mittels der Steuerdaten die Steuereinrichtung veranlasst wird, den Werkzeugaufsatz an die Führungsmarkervorrichtung heranzuführen. Anschließend wird der Verbindungsabschnitt mit der Fräskopfführung und/oder der Führungsmarkervorrichtung verbunden, insbesondere an diese angekoppelt. Es können auch Verbindungsdaten bestimmt werden, die Informationen darüber beinhalten, ob eine Verbindung des Verbindungsabschnitts mit der Fräskopfführung und/oder der Führungsmarkervorrichtung erfolgt ist oder nicht. Die Bestimmung der Verbindungsdaten kann insbesondere mittels der Führungsdaten und/oder Lagedaten und/oder Steuerdaten erfolgen. Insbesondere wenn die Verbindungsdaten die Verbindung anzeigen, wird die Bearbeitung des Knochens mit dem Werkzeugaufsatz freigegeben; bevor die Verbindung angezeigt wird, wird die Bearbeitung insbesondere blockiert. Im weiteren Verlauf des Verfahrens kann dann die räumliche Lage des Werkzeugaufsatzes nach dem Verbinden des Verbindungsabschnitts mit der Fräskopfführung und/oder der Führungsmarkervorrichtung erfasst werden. Diese Lageerfassung kann beispielsweise in einem Koordinatensystem erfolgen, das vor dem Verbinden fest bezüglich der Position des Werkzeugaufsatzes oder des Knochenteils war.

Zur Übermittlung der Steuersignale zwischen dem Steuerprogramm bzw. der Datenverarbeitungsvorrichtung und der Steuereinrichtung (die z.B. einen Roboterarm umfassen kann) ist es möglich, die Datenverarbeitungsvorrichtung durch ein Kabel mit dem Roboter zu verbinden. Eine Übermittlung der Steuersignale in Form optischer Signale und/oder Funksignale ist aber erfindungsgemäß ebenfalls möglich. Anschließend steuert das Steuerprogramm eine Steuereinrichtung (die mit der ersten Steuereinrichtung identisch sein kann, aber nicht sein muss, jedenfalls einen Roboter bzw. Roboterarm umfassen kann) derart an, dass ein oben beschriebener Werkzeugaufsatz an die Führungsmarkervorrichtung angelegt wird. Dies kann dadurch geschehen, dass die Längsachse des Werkzeugaufsatzes bzw. Roboterarms (wenn beispielsweise der Stützabschnitt des Werkzeugaufsatzes ortsfest relativ zum Roboterarm am Roboterarm angebracht ist) in einer geeigneten Position relativ zu der Längsachse des Oberschenkelkopfes ausgerichtet und an den Oberschenkelkopf heranbewegt bzw. in eine bestimmte, zum Fräsen geeignete Position (Bearbeitungsposition) relativ zum Oberschenkelkopf navigiert wird. Es ist auch möglich, den Werkzeugaufsatz so zu navigieren, dass eine Längsachse der spanabhebenden Teile (also des wenigstens einen Bearbeitungsabschnitts) eine bestimmte Position relativ zur Längsachse des Oberschenkelkopfes einnehmen, wobei die Längsachse des Bearbeitungsabschnitts in dieser Position insbesondere auf der und/oder parallel zur Längsachse des Oberschenkelkopfes liegt. Die Längsachse des wenigstens einen Bearbeitungsabschnitts ist insbesondere die Achse der Rotationssymmetrie, um die sich der wenigstens eine Bearbeitungsbaschnitt bei der vorzugsweise rotierenden Knochenbearbeitung bewegt. Bei der Annäherung des Werkzeugaufsatzes an den Oberschenkelkopf, insbesondere an die Führungsmarkervorrichtung erfolgt dann das Einführen der Führungsmarkervorrichtung in die Aussparung und/oder das Ankoppeln des Werkzeugaufsatzes an die Fräskopfführung , insbesondere deren Anschlußstück mittels des Verbindungsabschnitts.

In dem oben beschriebenen Steuerprogramm kann ein Programmabschnitt eingebaut werden, der auf die Notwendigkeit einer Entfernung der Führungsmarkervorrichtung hinweist. Dieser Programmabschnitt kann vorteilhaft nach dem Ankoppeln des Werkzeugaufsatzes an die Fräskopfführung ablaufen. Mit dem Entfernen der Führungsmarkervorrichtung ist das Entfernen vom Oberschenkelkopf bzw. von der Fräskopfführung (insbesondere von deren Anschlussstück) bzw. aus dem Arbeitsbereich (Bewegungsbereich) des wenigstens einen Bearbeitungsabschnitts gemeint. Der Hinweis wird vorteilhaft dann ausgegeben, wenn das chrirurgische Navigationssystem die Führungsmarkervorrichtung detektiert, obwohl sie gemäß einem vorbestimmten Programmablauf von ihrer Position im Arbeitsbereich des wenigstens einen Bearbeitungsabschnitts entfernt worden sein sollte und/oder die Markervorrichtung an einer Position detektiert, an der sie sich bei Beginn der Bearbeitung nicht befinden sollte.

Zu diesem Zweck können von dem Steuerprogramm Überprüfungsdaten ermittelt werden, die Informationen darüber beinhalten, ob wenigstens ein Teil der Führungsmarkervorrichtung und/oder der Knochennlarkervorrichtung (insbesondere aus dem Bewegungsbereich des wenigstens einen Bearbeitungsabschnitts) entfernt worden ist. In dem Steuerprogramm können ferner Hinweisinformationsdaten ermittelt, die eine Hinweisinformationsvorrichtung veranlassen, ein Signal (das insbesondere den Hinweis umfasst) auszugeben, das auf den Überprüfungsdaten basiert.

Der Hinweis kann als Signal, beispielsweise als visuelles und/oder akustisches und/oder taktiles Signal über eine Hinweisinformationsvorrichtung an einen Anwender ausgegeben werden. Dazu kann das Steuerprogramm ein Signal an eine visuelle Anzeigeeinrichtung, beispielsweise einen Monitor (der an die Datenverarbeitungseinrichtung bzw. das chirurgische Navigationssystem angeschlossen sein kann) ausgeben. Erfindungsgemäß möglich ist auch die Ausgabe eines Signals an eine an den Computer bzw. das chirurgische Navigationssystem angeschlossene akustische Signaleinrichtung, beispielsweise einen Lautsprecher bzw. eine Sprachmodulausgabevorrichtung, möglich. Die Hinweisinformation kann den Anwender darauf hinweisen, dass in dem entsprechenden Programmschritt eine Aktion des Anwenders notwendig ist, um die Führungsmarkervorrichtung bzw. die Markerhalter und/oder Referenzsternhalterung zu entfernen. Wenn die Führungsmarkervorrichtung die Fräskopfführung selbst durch z.B. stoffschlüssige Verbindung beinhaltet, wird somit wenigstens ein Teil der Markervorrichtung (nämlich die Markerhalter und/oder ein Haltelelement sowie die Markerelemente, nicht jedoch ein in der Fräskopfführung beinhalteter Befestigungsschaft samt Anschlussstück) entfernt. Eine Hinweisinformation kann auch entsprechend ausgegeben werden, wenn festgestellt worden ist, dass die Führungsmarkereinrichtung bereits entfernt worden ist. Die Feststellung, ob sich die Führungsmarkereinrichtung an der Fräskopfführung und/oder im Arbeitsbereich des Bearbeitungsabschnitts befindet oder nicht, kann z.B. über ein elektrisches Signal erfolgen: zwischen Markereinrichtung und Anlegemöglichkeit kann eine elektrische Spannung angelegt werden, die auf das Vorhandensein der Markereinrichtung hinweist. Der gemessene Spannungswert bzw. Strom kann als digitaler Messwert zur weiteren Verwendung an das Steuerprogramm weitergegeben werden. Eine analoge Abfrage und Hinweisinformation kann für die Knochenmarkervorrichtung vorgesehen sein, um ebenfalls sicherzustellen, dass diese vor der Bearbeitung aus dem Arbeitsbereich entfernt worden ist.

Das oben beschriebene Steuerprogramm kann auf einem computerlesbaren Medium wie einem magnetischen Speicher (z.B. einer Diskette, einer Festplatte, einem USB-Speicher bzw. Flashspeicher) und/oder einem optischen Speichermedium (wie einer CD-ROM oder DVD) gespeichert werden. Das computerlesbare Medium kann dann auf einem Computer ausgelesen werden, so dass das Steuerprogramm auf dem Computer abläuft.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speichermedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisung ausführt, verkörpert sind bzw. ist. Im Rahmen dieser Erfindung kann ein auf einem Computer verwendbares oder computerlesbares Medium irgendein Medium sein, dass das Programm zur Verwendung auf oder in Verbindung mit dem System, Apparat oder der Einrichtung, das bzw. der bzw. die die Anweisung ausführt, das Programm beinhalten, speichern, übermitteln, fortpflanzen oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise prozessiert werden könnte. Das Computerprogrammprodukt und jegliche Software und/oder Hardware, das bzw. die hier beschrieben werden, bilden in den beispielhaften Ausführungsformen die verschiedenen Mittel zum Ausführen der Funktionen der Erfindung.
Figur 1 zeigt das Registrieren eines Oberschenkelkopfes.
Figur 2 zeigt das auf einem Morphingverfahren beruhende Modell.
Figur 3a zeigt die Navigation einer Fräskopfführung relativ zu einem Oberschenkelkopf.
Figur 3b zeigt das Einführen einer Fräskopfführung in einen Oberschenkelkopf mit Hilfe einer Steuereinrichtung und eines chirurgischen Navigationssystems.
Figur 4 zeigt die Navigation eines an einer Steuereinrichtung befestigten erfindungsgemäßen Werkzeugaufsatzes relativ zu einer in einem Oberschenkelkopf befestigten Fräskopfführung.
Figur 5 zeigt einen erfindungsgemäßen Werkzeugaufsatz mit einer daran angeordneten Ankopplungsmöglichkeit.
Figur 6 zeigt einen erfindungsgemäßen Werkzeugaufsatz, der sich in einer Bearbeitungsposition an einem Oberschenkelkopf befindet.
Figur 7 zeigt einen erfindungsgemäßen Werkzeugaufsatz, der sich in einem Bearbeitungszustand an einem Oberschenkelkopf befindet.
Figur 8 zeigt ein Ablaufdiagramm eines öffnungsgemäßen Steuerprogramms zum Steuern des Werkzeugaufsatzes.

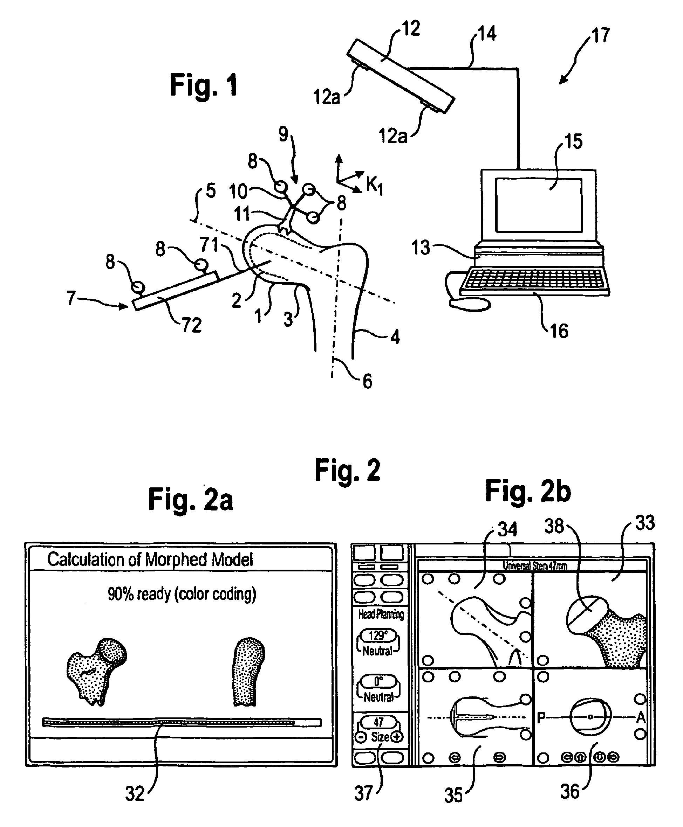

In Figur 1 wird ein Abschnitt eines Oberschenkelknochens mit einem Knochenschaft 4, einem Oberschenkelkopf 1 und einem Schenkelhals 3 gezeigt. Der Oberschenkelkopf 1 soll mit Hilfe eines erfindungsgemäßen Werkzeugaufsatzes 26 in die zu erzeugende Form 2 (die Bearbeitungsform 2) gebracht werden. Der Schaft 4 des Oberschenkelknochens weist eine Längsachse 6 auf, der Oberschenkelkopf 1 weist eine Längsachse 5 auf, die mit der Längsachse der Bearbeitungsform 2 vorteilhaft übereinstimmt. An dem Oberschenkelkopf 1 ist eine Markervorrichtung (Knochenmarkervorrichtung) 9 mit Markern 8, Markerhaltern 10 und einem Halteelement 11 angebracht. Ein Pointer 7 mit einem Haltegriff 72 und zwei Markern 8 wird verwendet, um die Geometrie der zu bearbeitenden ursprünglichen Oberfläche des Oberschenkellkopfes 1 abzugreifen bzw. zu registrieren. Dazu wird die Spitze 71 des Pointers (Pointerspitze) entlang der Oberfläche geführt. Ein chirurgisches Navigationssystem 17 umfasst einen Sender, insbesondere Infrarotsender 12 und zwei Empfänger, insbesondere zwei für elektromagnetische Strahlung, insbesondere Infrarotlicht empfindliche Erfassungseinrichtungen 12a (z.B. dafür empfindliche optische und/oder digitale Kameras), eine Datenverarbeitungsvorrichtung 13 (z.B. einen handelsüblichen Computer mit wenigstens einem Prozessor, einem Arbeitsspeicher und einem permanenten Speicher), einer Datenverbindung 14 (z.B. einer Funkverbindung bzw. Verbindung, die Daten mit Hilfe elektromagnetischer Strahlung übermittelt und/oder ein herkömmliches Datenkabel) zum Übermitteln von Daten zwischen dem Sender 12 und dem Empfängern 12 und der Datenverarbeitungsvorrichtung 13, eine Anzeigeeinrichtung 15 (die z.B. einen Röhrenmonitor oder einen TFT-Bildschirm und/oder einen Audiosender, etwa einen Lautsprecher, umfasst) zum Anzeigen von Daten, und Eingabemittel 16 (z.B. eine Tastatur und/oder eine Maus). Das chirurgische Navigationssystem 17 erfasst die Lage der Marker 8 am Pointer 7. Dadurch kann beim Abgreifen der ursprünglichen, im Rahmen des Verfahrens zu bearbeitenden Oberfläche des Oberschenkelkopfes 1 ein Modell der Oberflächengeometrie errechnet werden und dieses Modell im Koordinatensystem K₁, das z.B. das Raumkoordinatensystem in einem Behandlungsraum bzw. Operationssaal darstellt, eingeordnet werden.

In Figur 2 wird eine Bildschirmausgabe eines in einem Registrier- bzw. Morphingverfahren errechneten patientenspezifischen Modells einer Oberflächengeometrie eines Oberschenkelkopfes gezeigt. Das Anpassen eines generischen Knochenmodells mithilfe der registrierten Punkte des realen Knochens zu einem patienten-spezifischen Knochenmodell wird in einem so genannten Morphingverfahren durchgeführt. Figur 2a zeigt dabei verschiedene Perspektiven einer äußeren Ansicht des Oberflächenmodells. Ein Balken 32 zeigt dabei den Fortschritt der Berechnung des Modells an. Im gezeigten Fall ist die Berechnung des Oberflächenmodells zu 90 Prozent vollendet. Figur 2b zeigt die Bildschirmansicht einer Software, die auf der Datenverarbeitungsvorrichtung 13 laufen kann und zur Durchführung des erfindungsgemäßen Steuerprogramms beiträgt. Die Bildschirmausgabe 33 zeigt eine frontale oder dorsale Ansicht eines Oberschenkelkopfes 1, in der ein Bearbeitungsbereich 38 gekennzeichnet ist. Der Bearbeitungsbereich 38 stellt dabei den Bereich des Oberschenkelkopfes 1 dar, der mit dem erfindungsgemäßen Werkzeugaufsatz 26 bearbeitet, insbesondere gefräst werden soll. Die Bildschirmausgabe 34 zeigt eine frontale oder dorsale Ansicht eines Oberschenkelknochens 4 mit der darin kenntlich gemachten Längsachse 5 des Oberschenkelkopfes 1. Bildschirmausgabe 35 zeigt eine basale oder kraniale Ansicht eines Oberschenkelkopfes 1 und Schenkelhalses 3 mit der darin kenntlich gemachten Längsachse 5 bis Oberschenkelkopfes 1. Bildschirmausgabe 36 zeigt eine Ansicht des Oberschenkelkopfes 1 entlang seiner Längsachse 5. Die Bildschirmausgabe 37 umfasst Informationsfelder und Steuerungsfelder für einen Benutzer. Mit Hilfe der Informationsfelder wird der Benutzer über die Perspektive der Bildschirmausgaben 33, 34, 35, 36 informiert, beispielsweise erhält er Daten über einen Winkel, unter dem die Projektion des errechneten Oberflächenmodells erfolgt. Die Steuerungsfelder ermöglichen dem Benutzer, die Bildschirmausgaben 33, 34, 35, 36 so zu steuern, dass er die Perspektive der Darstellung für das Oberflächenmodell beeinflusst bzw. ändern kann.

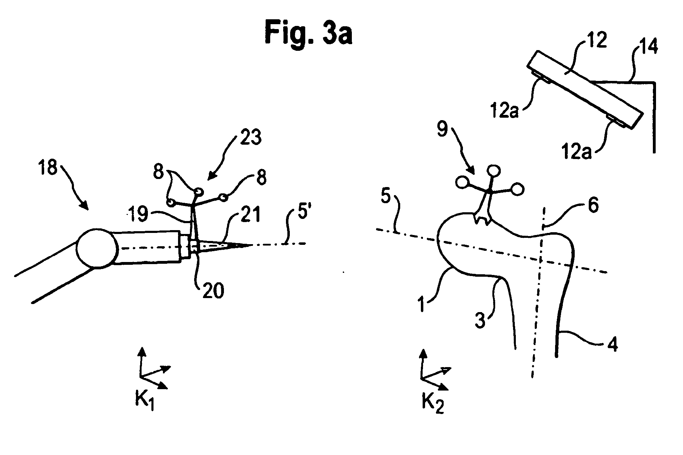

Figur 3a zeigt das Navigieren einer Fräskopfführung, die hier beispielsweise in Form eines Befestigungsschafts 21 ausgebildet ist, relativ zu einem Oberschenkelknochen 4, insbesondere einem Oberschenkelkopf 1, mit Hilfe einer Steuereinrichtung, die hier die Gestalt eines Roboterarmes 18 annimmt. Während der Navigation des Befestigungsschafts 21, an dessen Anschlussstück 20 eine Führungsmarkervorrichtung 23 mit Markern 8 und einem Halteelement 19 angebracht ist, relativ zum Oberschenkelkopf 1, an dem die oben beschriebene Knochenmarkervorrichtung 9 angebracht ist, wird der Befestigungsschaft 21 mit Hilfe eines chirurgischen Navigationssystems 17 geführt, dessen Sender 12, Empfänger 12a und Datenverbindung 14 in Figur 3a dargestellt sind. Dabei wird der Roboterarm 18 mit Hilfe des chirurgischen Navigationssystems 17 derart angesteuert, dass der Befestigungsschaft 21, dessen Position relativ zur Führungsmarkervorrichtung 23 vorteilhaft ortsfest und bekannt ist, eine definierte Lage bzw. Position relativ zum Oberschenkelkopf 1 einnimmt, dessen Position mit Hilfe der Knochenmarkervorrichtung 9 ermittelt werden kann. Insbesondere wird der Roboterarm 18 so angesteuert, dass die Längsachse 5' des Befestigungsschafts 21 parallel zur und/oder in Deckung mit der Längsachse 5 des Oberschenkelkopfes 1 gebracht wird. Die Längsachse 5' des Befestigungsschafts 21 kann parallel und/oder in Deckung mit einer Längsachse wenigstens eines unmittelbar vor dem Befestigungsschaft 21 bzw. dessen Anschlussstück 20 liegenden Endglieds des Roboterarms 18 sein. Während der Bewegung des Befestigungsschafts 21 relativ zum Oberschenkelkopf 1 können für die Navigation von Befestigungsschaft 21 und Oberschenkelkopf 1 unterschiedliche Koordinatensysteme verwendet werden. Diese Koordinatensysteme können insbesondere rechtwinklig bzw. kartesisch und/oder dreidimensional angelegt sein. So kann beispielsweise der Befestigungsschaft 21 in einem Koordinatensystem K₁ geführt werden, dessen Mittelpunkt bzw. Ursprung beispielsweise in einem Ort hat, der in einem Bauteil des Roboterarms 18 oder der Führungsmarkervorrichtung 23 oder des Anschlussstücks 20 oder des Befestigungsschafts 21 liegt. Die Navigation des Befestigungsschafts 21 erfolgt dann über eine Berechnung der Position des Befestigungsschafts 21 im Koordinatensystem K₁ relativ zu beispielsweise den Empfängern 12a des chirurgischen Navigationssystems 17. Dabei ist vorteilhaft die Position die Lage des Befestigungsschafts 21 bzw. die Lage seiner Längsachse 5' im Koordinatensystem K₁, insbesondere relativ zum Ursprung des Koordinatensystems K₁, bekannt. Analog kann der Knochenschaft 4 bzw. der Oberschenkelkopf 1 als Bestandteil des Oberschenkelknochens 4 in einem Koordinatensystem K₂ navigiert werden, dessen Mittelpunkt bzw. Ursprung in einem Ort liegt, der von einem Bestandteil des Knochenschafts 4 oder des Schenkelhalses 3 oder des Oberschenkelkopfes 1 oder der Knochenmarkervorrichtung 9 umfasst wird. Die Navigation des Oberschenkelkopfes 1 im Koordinatensystem K₂ erfolgt dann beispielsweise über eine Erfassung der Lage des Oberschenkelkopfes 1 relativ zu den Empfängern 12a des chirurgischen Navigationssystems 17. Dabei ist vorteilhaft die Position des Oberschenkelkopfes 1 bzw. die Lage seiner Längsachse 5 im Koordinatensystem K₂, insbesondere relativ zum Ursprung des Koordinatensystems K₂, bekannt. Nach dem Einführen des Befestigungsschafts 21 in den Oberschenkelkopf 1 kann die Navigation sowohl des Oberschenkelkopfs 1 bzw. Knochens 4 als auch des Befestigungsschafts 21 und/oder des Roboterarms 18 mithilfe nur der Führungsmarkervorrichtung 23 erfolgen. Dabei können die Positionen bzw. Lagen sowohl des Oberschenkelkopfes 1 (Oberschenkelknochens 4) als auch des Befestigungsschafts 21 bzw. Roboterarms 18 in einem gemeinsamen Koordinatensystem angegeben werden, das auf dem Koordinatensystem K₁' oder dem Koordinatensystem K₂' basieren kann.

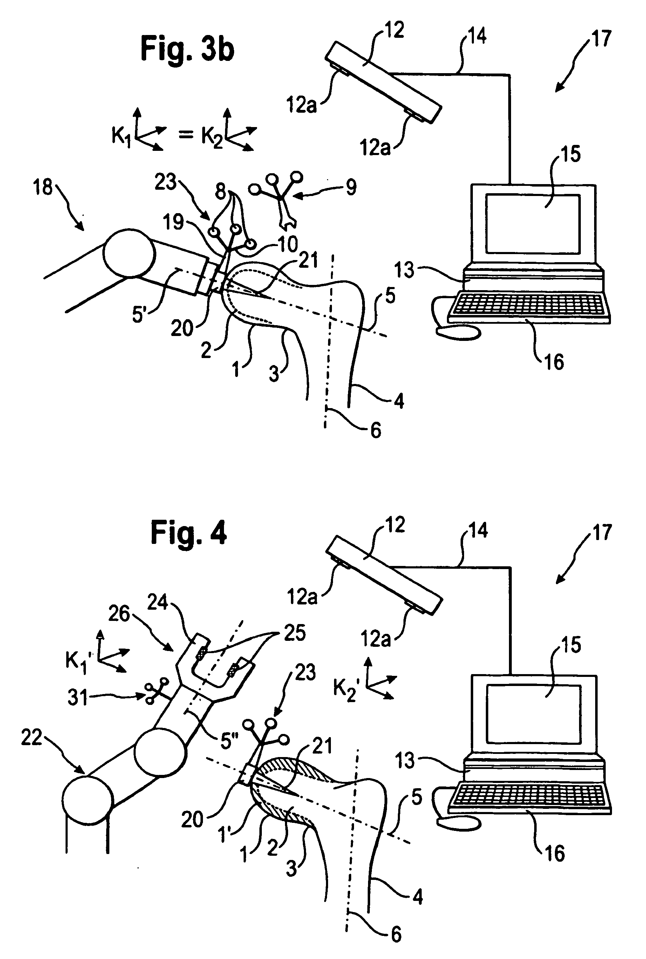

Figur 3b zeigt den Befestigungsschaft 21, wie er noch an dem Roboterarm 18 befestigt ist und bereits in den Oberschenkelkopf 1 eingeführt ist. Dabei befindet sich am Anschlussstück 20 noch die Führungsmarkervorrichtung 23. Da der Befestigungsschaft 21 und der Oberschenkelkopf 1 nun fest miteinander verbunden sind, beispielsweise über einen Schraubmechanismus und/oder eine formschlüssige und/oder kraftschlüssige Verbindung zwischen dem Befestigungsschaft 21 und dem Oberschenkelkopf 1, können der Befestigungsschaft 21 und der Oberschenkelkopf 1 nun in einem gemeinsamen Koordinatensystem navigiert werden. Dabei ist vorteilhaft die Position sowohl des Oberschenkelkopfes 1 als auch des Anschlussstücks 20 bzw. des Befestigungsschafts 21 relativ zum Ursprung des gemeinsamen Koordinatensystems bekannt. Für die Navigation der beiden Teile ist nun nur noch eine Führungsmarkervorrichtung 23 notwendig, so dass die Knochenmarkervorrichtung 9 vom Oberschenkelkopf 1 entfernt werden kann. Figur 3b zeigt ebenfalls, wie die Längsachse 5' des Befestigungsschafts 21 in Deckung mit der Längsachse 5 des Oberschenkelkopfs 1 gebracht werden kann. Nach dem Einführen des Befestigungsschafts 21 in den Oberschenkelkopf 1 kann der Roboterarm 18 vom Befestigungsschaft 21 bzw. dessen Anschlussstück 20 entfernt werden. Zweckmäßig ist es, die Führungsmarkervorrichtung 23 noch am Anschlussstück 20 befestigt zu belassen, damit der Werkzeugaufsatz später relativ zur Führungsmarkervorrichtung 23 (analog zu Navigation des Befestigungsschafts 21 relativ zum Oberschenkelkopf 1) navigiert werden kann.

Figur 4 zeigt einen in den Oberschenkelkopf 1 eingeführten Befestigungsschaft 21 mit einer an seinem Anschlussstück 20 angebrachten Führungsmarkervorrichtung 23. Relativ zum Anschlussstück 20, dessen Position relativ zur Führungsmarkervorrichtung 23 vorteilhaft ortsfest und bekannt ist, kann nun ein Werkzeugaufsatz 26 mit Hilfe eines chirurgischen Navigationssystems 17 navigiert werden. Der Werkzeugaufsatz 26 ist dabei vorteilhaft an einem Roboterarm 22 angebracht, der identisch sein kann mit dem Roboterarm 18, aber auch einen davon unterschiedlichen Roboterarm darstellen kann. Vorteilhaft ist an den Roboterarm 22 eine Markervorrichtung (Robotermarkervorrichtung) 31 angebracht, wobei insbesondere die Position des Werkzeugaufsatzes 26 relativ zur Robotermarkervorrichtung 31 bekannt ist. Eine andere Markervorrichtung (Werkzeugmarkervorrichtung) 31 kann zusätzlich oder alternativ an einen Teil des Werkzeugaufsatzes 26 angebracht sein, wobei auch dann die Position des Werkzeugaufsatzes 26 relativ zur Werkzeugmarkervorrichtung 31 vorteilhaft ortsfest und bekannt ist. Die Navigation des Roboterarms 18, 22 kann mithilfe einer Robotermarkervorrichtung 31 und/oder einer Werkzeugmarkervorrichtung 31 erfolgen. Erfindungsgemäß ist aber auch möglich, die Navigation des Roboterarms 18, 22 lediglich mithilfe der vorbekannten Dimensionen des Roboterarms 18, 22 bzw. Kenntnis der Lage seiner Bestandteile relativ zu einem vorbekannten Ort im Bezugssystem K₁ bzw. K₁' durchzuführen. Im letzteren Fall ist es möglich, zur Navigation des Roboterarms 18, 22 bzw. des Werkzeugaufsatzes 26 auf eine Robotermarkervorrichtung 31 bzw. Werkzeugmarkervorrichtung 31 zu verzichten. Der Werkzeugaufsatz 26 umfasst einen Stützabschnitt 24, der vorteilhaft rotationssymmetrische Form besitzt. So kann der Stützabschnitt 24 wie oben erläutert Zangenform aufweisen, aber auch in Form eines Hohlzylinders ausgebildet sein. Vorteilhaft befindet sich dabei in dem Stützabschnitt eine Öffnung in Richtung einer dem Roboterarm 22 abgewandten Seite des Werkzeugaufsatzes 26, so dass der Stützabschnitt 24 um einen Oberschenkenkopf 1 herum positioniert werden kann bzw. ein Oberschenkelkopf 1 in das Innere des Stützabschnitts 24 eingeführt werden kann. An dem Stützabschnitt 24 ist wenigstens ein Bearbeitungsabschnitt 25 angeordnet, wobei der Bearbeitungsabschnitt 25 insbesondere einen Bohrer und/oder eine Fräse beinhalten kann. Der Bearbeitungsabschnitt 25 ist im Falle einer Fräse insbesondere dazu ausgelegt, den schraffierten Bearbeitungsbereich 1' des Oberschenkelkopfes 1 so zu bearbeiten bzw. zu fräsen bzw. zu entfernen, dass eine Bearbeitungsform 2 des Oberschenkelkopfes, die insbesondere zylindrische Grundform aufweist, zum Ende (d.h. dem Ende, das anatomisch auf die Hüftgelenkspfanne weist) des Oberschenkelkopfes hin aber auch zugespitzt bzw. konusförmig verlaufen kann, zu erzeugen. Die Bewegung bzw. Navigation des Werkzeugaufsatzes 26 relativ zum Oberschenkelkopf 1 bzw. Anschlussstück 20 erfolgt wiederum mit Hilfe eines chirurgischen Navigationssystems 17 in Analogie zur Navigation des Roboterarms 18 bzw. Befestigungsschafts 21 relativ zum Oberschenkelkopf 1, wie sie in Figuren 3a und 3b dargestellt und oben erläutert wird. Die Position des Oberschenkelkopfes 1 relativ zur Führungsmarkervorrichtung 23 ist dabei vorteilhaft ortsfest und bekannt. Die Führung des Roboterarms 22 bzw. Werkzeugaufsatzes 26 kann in einem Koordinatensystem K₁' erfolgen, das unterschiedlich ist vom Koordinatensystem K₂', in dem der Oberschenkelkopf 1 bzw. das Anschlussstück 20 geführt wird. Die Koordinatensysteme K₁' und K₂' können wiederum rechtwinklig bzw. kartesisch und/oder dreidimensional angelegt sein. Der Ursprung des Koordinatensystems K₁' kann in einem Ort liegen, der von einem Bestandteil des Werkzeugaufsatzes 26 oder des Roboterarms 22 oder einer Robotermarkervorrichtung 31 oder Werkzeugmarkervorrichtung 31 umfasst wird. Der Ursprung des Koordinatensystems K₂' kann in einem Ort liegen, der vom Oberschenkelkopf 1 oder dem Befestigungsschaft 21 oder der Bearbeitungsform 2 oder dem Oberschenkelschaft 4 oder der Führungsmarkervorrichtung 23 oder dem Anschlussstück 20 umfasst wird. Vorteilhaft ist die Lage bzw. Position des Werkzeugaufsatzes 26 relativ zum Ursprung des Koordinatensystems K₂' bekannt. Vorteilhaft ist die Lage bzw. Position des Oberschenkelkopfes 1 bzw. des Anschlussstücks 20 relativ zum Ursprung des Koordinatensystems K₁' bekannt. Vorteilhaft wird der Werkzeugaufsatz 26 so relativ zum Oberschenkelkopf 1 bzw. Anschlussstück 20 navigiert bzw. bewegt, dass die Längsachse 5" des Werkzeugaufsatzes 26 parallel und/oder in Deckung mit der Längsachse 5 des Oberschenkelkopfes und/oder des Befestigungsschafts 21 positioniert wird. Dabei kann die Längsachse 5" des Werkzeugaufsatzes 26 parallel und/oder in Deckung mit einer Längsachse wenigstens eines unmittelbar vor dem Werkzeugaufsatz 26 angeordneten Endstücks des Roboterarms 22 sein. Nach dem Ankoppeln des Werkzeugaufsatzes 26 an den Befestigungsschaft 21 kann die Navigation sowohl des Werkzeugaufsatzes 26 als auch des Befestigungsschaftes 21 mithilfe nur der Führungsmarkervorrichtung 23 erfolgen. Die Navigation kann dabei so durchgeführt werden, dass die Positionen bzw. Lagen sowohl des Werkzeugaufsatzes 26 als auch des Befestigungsschaftes 21 in einem gemeinsamen Koordinatensystem bestimmt werden, das auf dem Koordinatensystem K₁' oder dem Koordinatensystem K₂' beruhen kann.

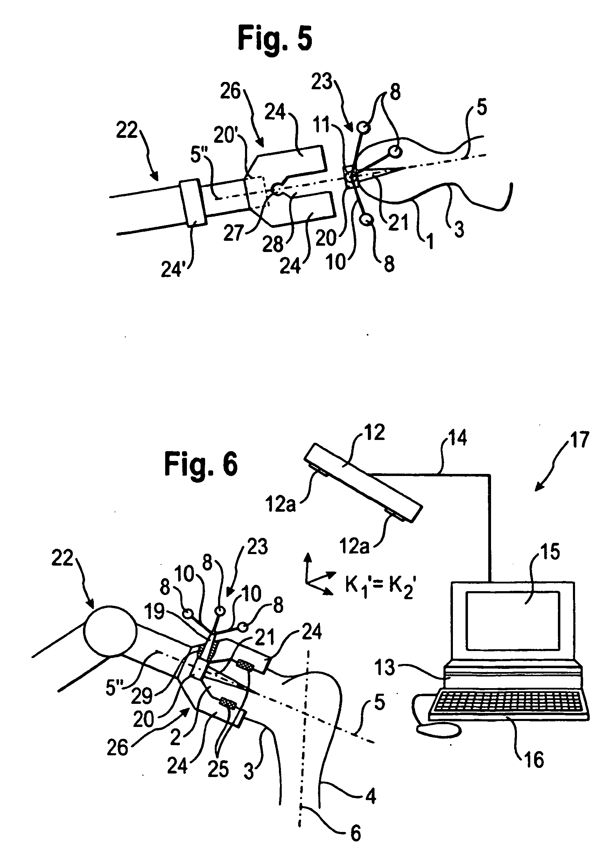

Figur 5 zeigt eine perspektivische Ansicht in basaler oder kranialer Richtung des Oberschenkelkopfs 1 mit einem darin eingeführten Befestigungsschaft 21. Am Anschlussstück 20 des Befestigungsschafts 21 ist eine Führungsmarkervorrichtung 23 mit Markern 8 und Markerhaltern 10 angebracht. Der Werkzeugaufsatz 26, der sich an einem Roboterarm 22 befindet, ist nun so ausgerichtet, dass seine Längsachse 5" in Deckung mit der Längsachse 5 des Oberschenkelkopfes 1 bzw. Befestigungsschafts 21 gebracht ist. Der Werkzeugaufsatz 26 weist eine Aussparung 28 in Form eines Schlitzes (der insbesondere auf einer bzw. parallel zu einer Achse der Rotationssymmetrie bzw. einer Längsachse bzw. in einer Ebene liegt, die parallel zu einer solchen Achse verläuft) auf. Diese Aussparung 28 ist zwischen bzw. in Teilen des Stützabschnitts 24 angeordnet. Falls der Stützabschnitt 24 in Zangenform ausgebildet ist, befindet sich die Aussparung 28 zwischen den Zangenarmen des Stützabschnitts 24. Die Aussparung 28 weist Schlitzform auf und ist auf einer Seite, die dem Roboterarm 22 vorteilhaft abgewandt ist und in der dargestellten Position insbesondere auf das Anschlussstück 20 hinweist, offen, um das Einführen eines Halteelements 11 oder eines anderen Teils einer Führungsmarkervoirichtung 23 in den Werkzeugaufsatz 26 zu ermöglichen. Dabei kann das Halteelement 11 bzw. ein anderer Teil einer Führungsmarkervorrichtung 23 bis an das dem Roboterarm 22 zugewandten Ende der Aussparung 28 geführt werden und in ein Eingreifelement 27 eingelegt werden. Das Eingreifelement 27 kann insbesondere rund oder auch mehreckig ausgestaltet sein und für einen formschlüssigen und/oder kraftschlüssigen bzw. passgenauen Sitz des Werkzeugaufsatzes 26 an dem Halteelement 11 bzw. der Führungsmarkervorrichtung 23 sorgen. Wenn die Führungsmarkervorrichtung 23 nicht an dem Anschlussstück 20 oder an einer von einem Stützabschnitt 24 und/oder Bearbeitungsabschnitt 25 überdeckten Ort des Oberschenkelkopfs 21 angebracht ist, kann an dem Werkzeugaufsatz 26 eine Sonde vorgesehen sein, die eine mechanische und ortsfeste, insbesondere kraftschlüssige und/oder formschlüssige Verbindung zwischen dem Werkzeugaufsatz 26 und der Führungsmarkervorrichtung 23 ermöglicht. Die Aussparung 28 kann dann z.B. in Form eines Schlitzes an der Sonde angeordnet sein. Außerdem weist der Werkzeugaufsatz 26 einen Verbindungsabschnitt 20' auf, der eine stabile mechanische, insbesondere formschlüssige und/oder kraftschlüssige Verbindung mit dem Anschlussstück 20 eingehen kann. Die Befestigungsmöglichkeit 24' dient zum Anschließen des Werkzeugaufsatzes 26 an den Roboterarm 22.

Figur 6 zeigt den Werkzeugaufsatz 26 in einem an einem Oberschenkelkopf 1 angelegten Zustand. Dabei greift das Anschlussstück 20 in einen Verbindungsabschnitt des Werkzeugaufsatzes 26 ein, das vorteilhaft auf der Längsachse 5" des Werkzeugaufsatzes 26 angeordnet ist. Die mechanische Verbindung zwischen dem Anschlussstück 20 und dem Verbindungsabschnitt kann formschlüssig und/oder kraftschlüssig ausgebildet sein. Der Stützabschnitt 24 umlagert den Oberschenkelkopf 1 so, dass die Bearbeitungsabschnitte 25 an den Bearbeitungsbereich 1' wenigstens teilweise anliegen bzw. diesem gegenüberliegen. Das Eingreifelement 27 ist vorteilhaft so angeordnet, dass es die Führungsmarkervorrichtung 23 umgreift, wenn der Werkzeugaufsatz 26 sich in einer Bearbeitungsposition in dem Oberschenkelkopf 1 befindet.

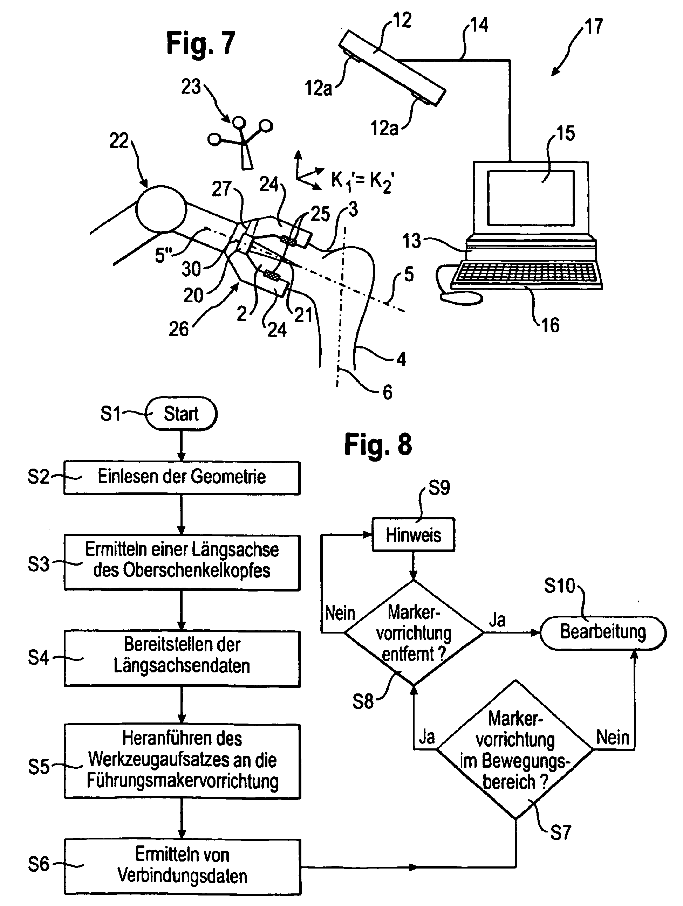

In Figur 7 wird gezeigt, dass die Führungsmarkervorrichtung 23 zur Bearbeitung des Bearbeitungsbereichs 1' vom Werkzeugaufsatz 26 entfernt wird. Der Werkzeugaufsatz 26 befindet sich damit in einer Bearbeitungsposition, d.h. er hat in seiner Bearbeitungskonfiguration einer Bearbeitung geeignete Position am zu bearbeitenden Knochenteil eingenommen. Dabei kann eine Öffnung in dem Werkzeugaufsatz 26, die insbesondere durch das Eingreifelement 27 gebildet wird, vorhanden bleiben. Erfindungsgemäß ist aber auch möglich, das Eingreifelement 27 dann so zu verschließen, dass keine Partikel von außen in das Innere des Werkzeugaufsatzes bzw. an das Verbindungsteil 20 gelangen können. Das Verschließen des Eingreifelements 27 ist beispielsweise mit einer Kappe aus einem Kunststoff oder einem Metall möglich, die formschlüssig und/oder kraftschlüssig von außen auf die Öffnung des Eingreifelements 27 aufgesetzt wird. Auf analoge Art ist es möglich, den Schlitz der Aussparung 28 vor der Bearbeitung des Bearbeitungsbereichs 1' zu verschließen. So kann beispielsweise eine Verschmutzung des Arbeitsbereichs bei Bewegung bzw. Rotation der Bearbeitungsabschnitte 25 und/oder des Stützabschnitts 24 verringert bzw. vermieden werden. Bei der Bearbeitung des Bearbeitungsbereichs 1' kann der Stützabschnitt 24 ortsfest relativ zum Roboterarm 22 und/oder Oberschenkelkopf 1 bleiben, er kann sich aber auch relativ zum Roboterarm 22 bewegen, insbesondere entlang seiner Längsachse 5" rotieren, um eine rotierende Fräsbearbeitung des Bearbeitungsbereichs 1' zu ermöglichen. In diesem Fall ist es vorteilhaft, vor der Bearbeitung des Knochens die Führungsmarkervorrichtung 23 zu entfernen, um eine Beschädigung des Werkzeugaufsatzes 26 und/oder der Führungsmarkervorrichtung 23 zu vermeiden. In diesem Fall ist es möglich, die Führungsmarkervorrichtung 23 an ihrem Ort, d.h. insbesondere an dem Anschlussstück 20 bzw. am Eingreifelement 27 zu belassen. Dies ist vor allem dann möglich, wenn die Führungsmarkervorrichtung 23 sich in dieser Position außerhalb eines Bewegungsbereiches von zur Bearbeitung der Knochenoberfläche bewegten Teilen des Werkzeugaufsatzes 26 (d.h. insbesondere außerhalb des Bewegungsbereiches der Bearbeitungsabschnitte 25) befindet.

Figur 8 zeigt ein Ablaufdiagramm für das erfindungsgemäße Datenverarbeitungsverfahren, nämlich das Steuerprogramm zum Positionieren eines oben beschriebenen Werkzeugaufsatzes 26 an einem Knochenteil, insbesondere einem Oberschenkelkopf 1, mittels einer Steuereinrichtung. Die Steuereinrichtung kann dabei ein chirurgisches Navigationssystem 17 und/oder einen Roboterarm 18, 22 umfassen. In einem Schritt Slwird das Programm gestartet, beispielsweise von der Datenverarbeitungsvorrichtung 13 aufgerufen. Zunächst wird in einem Schritt S2 die Geometrie des Oberschenkelkopfes 1 insbesondere mit Hilfe eines Registrierverfahrens eingelesen. Möglich ist aber auch die Verwendung von Geometriedaten aus einem bildgebenden Verfahren (insbesondere MRT oder CT). Aus der Geometrie des Oberschenkelkopfes bzw. dem ermittelten Modell des Oberschenkelkopfes wird eine Längsachse 5 des Oberschenkelkopfes 1 ermittelt. Anschließend kann mit einem Roboterarm 18 eine Führungsmarkervorrichtung 23 auf der Längsachse 5 des Oberschenkelkopfs 1 angebracht werden. Diese Führungsmarkervorrichtung 23 kann insbesondere auf einem Verbindungsteil 20 eines Befestigungsschafts 21 angebracht sein. Die Führungsmarkervorrichtung 23 kann aber auch an einer anderen Stelle des Oberschenkelkopfes angebracht werden, die voiteilhaft in einer ortsfest und bekannten Lage relativ zu einem Oberschenkelkopf 1 eingeführten Befestigungsschaft 21 bzw. dessen Anschlussstück 20 liegt. In einem Schritt S4 werden Längsachsendaten bereitgestellt, die die relative Lage zwischen der Führungsmarkervorrichtung und der Längsachse 5 beschreiben. So dann wird in einem Schritt S5 der Werkzeugaufsatz 26 relativ zur Führungsmarkervorrichtung 23 navigiert, insbesondere an diese herangeführt. Dazu werden insbesondere die oben beschriebenen Lagedaten dem Steuerprogramm bereitgestellt. Im Schritt S6 ermittelt das Steuerprogramm Verbindungsdaten, die insbesondere Informationen darüber beinhalten, ob der Werkzeugaufsatz 26 (insbesondere dessen Verbindungsabschnitt 20') mit dem Befestigungsschaft 21 und/oder der Führungsmarkervorrichtung 23 mechanisch verbunden ist. Ferner können dem Steuerprogramm insbesondere vom chirurgischen Navigationssystem 17 Typdaten bereitgestellt werden, die Informationen über den Typ des benutzten Werkzeugaufsatzes 26 beinhalten. Dies kann beispielsweise dadurch geschehen, dass in dem Werkzeugaufsatz 26 ein magnetischer Datenspeicher mit den entsprechenden, darauf abgespeicherten Informationen vorgesehen ist, der über den Roboterarm 22 von der Datenverarbeitungsvorrichtung 13 ausgelesen werden kann. Dazu ist der Roboterarm 22 mit der Datenverarbeitungsvorrichtung 23 über eine Datenverbindung 14 verbunden. Die Datenverarbeitungsvorrichtung 13 kann Daten aus dem magnetischen Speicher auslesen, die Informationen über den Typ des Werkzeugaufsatzes und dessen Betriebseigenschaften und/oder Geometrie umfassen. Gegebenenfalls kann die Datenverarbeitungsvorrichtung 13 auch nur Daten über den Typ des Werkzeugaufsatzes erhalten und mit einer im permanenten Speicher der Datenverarbeitungsvorrichtung 13 abgelegten Datensatz über den Typ des Werkzeugaufsatzes 26 vergleichen. Dieser Vergleich kann ein Ergebnis liefern, das Informationen über die Betriebseigenschaften wie z.B. den Bewegungsbereich eines Bearbeitungsabschnitts 25 bei der Bearbeitung des Bearbeitungsbereichs 1' beinhaltet. Diese Daten können mit der vom chirurgischen Navigationssystem 17 erfassten Position der Führungsmarkervorrichtung 23 verglichen werden. In einem Schritt S7 wird dementsprechend überprüft, ob die Führungsmarkervorrichtung 23 sich im Bewegungsbereich eines zur Bearbeitung des Bearbeitungsbereichs 1' bewegten Teils (insbesondere eines Bearbeitungsabschnitts 25) des Werkzeugaufsatzes 26 befindet. Ist dies nicht der Fall, wird in einem Schritt S 10 mit der Bearbeitung des Bearbeitungsbereichs 1' fortgefahren. Befindet sich die Markervorrichtung jedoch im Bewegungsbereich gemäß Schritt S7, wird eine Überprüfung in einem Schritt S8 durchgeführt, ob die Führungsmarkervorrichtung 23 aus dem Bewegungsbereich entfernt worden ist. Es werden also Überprüfungsdaten ermittelt, die Informationen darüber umfassen, ob wenigstens ein Teil der Führungsmarkervorrichtung 23 und/oder der Knochenmarkervorrichtung 9 entfernt worden ist bzw. sich kein Teil der Führungsmarkervorrichtung 23 und/oder der Knochenmarkervorrichtung 9 noch im Bewegungsbereich befindet. Die Ermittlung der Überprüfungsdaten kann insbesondere aus einem Vergleich der Lage der Führungsmarkervorrichtung 23 und/oder der Knochenmarkervonichtung 9 sowie des Werkzeugaufsatzes 26 erfolgen. Aufgrund der Überprüfungsdaten wird dann in einem Schritt S10 entschieden, ob mit einer Bearbeitung des Knochenteils 1 begonnen werden kann. Die Überprüfungsdaten können auch derart ermittelt werden, dass ein Benutzer eine manuelle Eingabe in die Datenverarbeitungsvorrichtung vornimmt, die Informationen darüber an die Datenverarbeitungsvorrichtung weitergibt, ob die Führungsmarkervorrichtung 23 aus dem Bewegungsbereich entfernt worden ist oder nicht. Diese Eingabe kann dann die Ermittlung der Überprüfungsdaten vereinfachen, indem der Vergleich der Lagen von Werkzeugaufsatz 26 und Führungsmarkervorrichtung 23 und/oder Knochenmarkervorrichtung 9 entbehrlich wird. Falls die Führungsmarkervorrichtung 23 aus dem Bewegungsbereich entfernt worden ist, wird in einem Schritt S10 mit der Bearbeitung eines Bearbeitungsbereichs 1' fortgefahren. Ist das Ergebnis von Schritt S8 negativ, d.h. ist die Führungsmarkervorrichtung 23 nicht aus dem Bewegungsbereich entfernt, erfolgt in einem Schritt S9 ein Hinweis an einen Benutzer darüber, dass die Führungsmarkervorrichtung 23 sich im Bewegungsbereich befindet. Dieser Hinweis kann beispielsweise auf einer Ausgabevorrichtung 15 in visueller Form vorgenommen werden, es ist erfindungsgemäß aber auch möglich den Hinweis als Audiosignal (z.B. als Sprachausgabe oder einfaches akustisches Warnsignal) auszugeben. Nach Ausgabe des Hinweises kehrt das Steuerprogramm zu Schritt S8 zurück, in dem wiederum überprüft wird, ob die Führungsmarkervorrichtung 23 inzwischen aus dem Bewegungsbereich entfernt worden ist. Die Entscheidung gemäß Schritt S8, ob die Führungsmarkervorrichtung 23 aus dem Bewegungsbereich entfernt ist, kann über einen Vergleich der vom chirurgischen Navigationssystem 17 erfassten Position der Führungsmarkervorrichtung 23 und der in Schritten S6 und S7 ermittelten Betriebseigenschaften des Werkzeugaufsatzes 26 erfolgen. Ist die Führungsmarkervorrichtung 23 nicht im Bewegungsbereich ist (nein in Schritt S7) oder die Führungsmarkervorrichtung aus dem Bewegungsbereich entfernt worden (ja in Schritt S8), kann in einem Schritt S10 mit der Bearbeitung begonnen werden.

Anschließend kann von dem Steuerprogramm die Steuerung des Werkzeugaufsatzes 26 freigegeben werden. Die Entscheidung, ob der Werkzeugaufsatz 26 freigegeben wird oder nicht, kann insbesondere auf dem Ergebnis von Schritt S8 basieren. Unter Freigeben des Werkzeugaufsatzes 26 wird hierbei insbesondere verstanden, dass der Werkzeugaufsatz 26 keine Steuersignale mehr vom Steuerprogramm empfängt, die seine räumliche Position beeinflussen. Möglich ist aber erfindungsgemäß, dass weiterhin Steuersignale an den Werkzeugaufsatz 26 gesandt werden, die eine räumlich eingegrenzte Bewegung bzw. Lageveränderung des Werkzeugaufsatzes ermöglichen. Dadurch wird gewährleistet, dass während der Bearbeitung der Werkzeugaufsatz seine Position minimal verändern kann, insbesondere mitschwingen kann, um erhöhte mechanische Spannung und dadurch eine Schädigung des Knochenteils 1 zu vermeiden bzw. weniger wahrscheinlich zu machen. Der Werkzeugaufsatz 26 kann aber auch derart freigegeben werden, dass seine Möglichkeit zur Lageveränderung nur noch durch die geometrischen Dimensionen des Roboterarms 22, insbesondere dessen Bewegungsfreiheit, eingeschränkt werden. Vorteilhaft werden aber in diesem Verfahrensschritt keine Steuersignale mehr an den Roboterarm 22 gesendet, die eine aktive Lageveränderung, insbesondere eine Lageveränderung, die auf einen Antrieb des Roboterarms 22 zurückzuführen sind, bewirken. Unter einem passiven Steuersignal, das an den Werkzeugaufsatz 26 gesandt wird, wird in diesem Zusammenhang insbesondere ein Steuersignal verstanden, das erst dann ausgesandt wird, wenn der Werkzeugaufsatz 26 eine bestimmte Lage einnimmt bzw. ein bestimmtes Bewegungsmuster ausführt. Ein passives Steuersignal wird also als Reaktion auf eine Bewegung bzw. Lageveränderung des Werkzeugaufsatzes 26 und/oder in Reaktion auf Kräfte, die auf den Werkzeugaufsatz 26 und/oder Roboterarm 22 wirken (z.B. mit Beschleunigungssensor, Drucksensor, oder Kraftsensor) detektiert wurden, und als Detektionssignale dem Steuerprogramm zugeführt wurden, ausgesandt und veranlasst den Roboterarm 22 insbesondere dazu, diese Bewegung bzw. Lageänderung nicht (vollständig) zu unterdrücken oder zu blockieren, um durch die Bewegung erzeugte Kräfte zwischen dem Knochenteil 1 und dem Werkzeugaufsatz zu minimieren, reduzieren oder vollständig zu unterdrücken. Insbesondere wird einer Bewegung des Werkzeugaufsatzes 26 bzw. Kräften auf den Werkzeugaufsatz 26 und/oder den Roboterarm 22 entgegengewirkt. Die Bewegung bzw. Kräfte können somit gedämpft werden.

Schließlich können an den Werkzeugaufsatz 26 Bearbeitungsdaten bzw. Bearbeitungssignale gesendet werden, die Information darüber beinhalten, dass mit der Bearbeitung, insbesondere dem Fräsen des Knochenteils 1 begonnen wird bzw. werden soll. Diese Signale bzw. Daten haben vorteilhaft die Form eines Impulses, der ein insbesondere vorbestimmtes Antriebsschema des wenigstens einen Bearbeitungsabschnitts 25 ablaufen lässt. Die Bearbeitungsdaten können aber auch weitere Informationen beinhalten, z.B. Informationen darüber, mit welcher Kraft und/oder Geschwindigkeit und/oder in welchem Muster das Knochenteil 1 zu bearbeiten ist. Insbesondere bewirken die Bearbeitungsdaten also, dass der Werkzeugaufsatz 26 mit der Bearbeitung des Knochenteils 1 beginnt. Die Bearbeitungsdaten zeigen insbesondere erst dann an, dass mit der Bearbeitung begonnen wird, wenn die Überprüfungsdaten anzeigen, dass die Führungsmarkervorrichtung 23 und/oder die Knochenmarkervorrichtung 9 entfernt worden ist und/oder wenn die Verbindungsdaten eine Verbindung des Werkzeugaufsatzes 26 mit der Werkzeugaufsatzführung 21 anzeigen.

## Patentansprüche

1. Werkzeugaufsatz (26) für medizintechnische Anwendungen, insbesondere zum Fräsen eines Knochenteils (1), insbesondere eines Oberschenkelkopfes, mit
• einem Verbindungsabschnitt (24), der während der Bearbeitung eines Knochenteils (1) mit dem Werkzeugaufsatz (26) relativ zum Knochenteil (1) ruht; und
• wenigstens einem Bearbeitungsabschnitt (25) zum Bearbeiten eines Knochenteils (1);
wobei
der Verbindungsabschnitt (24) zum ortsfesten Verbinden des Werkzeugaufsatzes mit einer Werkzeugaufsatzführung (21) ausgebildet ist,
**dadurch gekennzeichnet, dass** der Werkzeugaufsatz eine Befestigungsmöglichkeit (24') zum Befestigen des Werkzeugaufsatzes (26) an einer Steuereinrichtung (22) aufweist, und dass
der Bearbeitungsabschnitt (25) eine Aussparung (28) aufweist, die ausgebildet ist, eine Führungsmarkervorrichtung (23) aufzunehmen und ein Verbinden des Verbindungsabschnitts (24) mit der Werkzeugaufsatzführung (21) zu ermöglichen.

2. Werkzeugaufsatz (26) nach Anspruch 1, **dadurch gekennzeichnet, dass** ein Stützabschnitt und/oder der Verbindungsabschnitt (24) die Aussparung (28) aufweist.

3. Werkzeugaufsatz (26) nach Anspruch 1, **dadurch gekennzeichnet, dass** die Aussparung (28) eine Verbindung des Werkzeugaufsatzes (26) mit der Führungsmarkervorrichtung (23) unterstützt.

4. Werkzeugaufsatz (26) nach einem der vorhergehenden Ansprüche, **dadurch gekennzeichnet, dass** die Lage der Aussparung (28) relativ zum Werkzeugaufsatz (26) verstellbar ist.

5. Werkzeugaufsatz (26) nach einem der Ansprüche 1 bis 4, **dadurch gekennzeichnet, dass** die Aussparung (28) ein Eingreifelement (27) beinhaltet, das eine mechanische Verbindung zwischen dem Werkzeugaufsatz (26) und der Führungsmarkervorrichtung (23) unterstützt.

6. System aus einem Werkzeugaufsatz (26) nach einem der vorhergehenden Ansprüche und einer Führungsmarkervorrichtung (23), wobei der Verbindungsabschnitt (24) ausgebildet ist, die Führungsmarkervorrichtung (23) aufzunehmen, um ein ortsfestes Verbinden des Werkzeugsaufsatzes (26) mit der Führungsmarkervorrichtung (23) zu ermöglichen.

7. System aus einem Werkzeugaufsatz (26) nach einem der Ansprüche 1 bis 5 oder einem System nach dem vorhergehenden Anspruch und einem chirurgischen Navigationssystem (17).

8. Steuerprogramm zum Positionieren des Werkzeugaufsatzes (26) nach einem der Ansprüche 1 bis 5 an einem Knochenteil (1), insbesondere einem Oberschenkelkopf, mittels einer Steuereinrichtung (22), das auf einer Datenverarbeitungsvorrichtung (13) abläuft und die folgenden Schritte umfasst:
• Bereitstellen (S4) von Führungsdaten, die Informationen über die Lage einer Führungsmarkervorrichtung (23) bezüglich des Knochenteils (1) umfassen;
• Bereitstellen von Lagedaten, die Informationen über die Lage des Werkzeugaufsatzes (26) umfassen;
• Bestimmen von Steuerdaten basierend auf den Führungsdaten und Lagedaten, wobei die Steuerdaten ausgebildet sind, die Steuereinrichtung (22) zu veranlassen, den Werkzeugaufsatz (26) an die Führungsmarkervorrichtung (23) heranzuführen (S5), so dass die Führungsmarkervorrichtung (23) in einer an dem Werkzeugaufsatz (26) vorgesehenen Aussparung (28) aufgenommen wird;
• Bestimmen (S6) von Verbindungsdaten basierend auf den Führungsdaten, Lagedaten und Steuerdaten, die Informationen über eine Verbindung eines Verbindungsabschnitts (24) des Werkzeugaufsatzes (26) mit der Werkzeugaufsatzführung (21) umfassen.

9. Steuerprogramm nach dem vorhergehenden Anspruch, das weiter folgenden Schritt umfasst:
Ermitteln (S8) von Überprüfungsdaten, die Informationen darüber umfassen, ob wenigstens ein Teil der Führungsmarkervorrichtung (23) und/oder der Knochenmarkervorrichtung (9) entfernt worden ist.

10. Steuerprogramm nach dem vorhergehenden Anspruch, das weiter folgenden Schritt umfasst:
Ermitteln von Hinweisinformationsdaten, die eine Hinweisinformationsvorrichtung veranlassen, ein Signal auszugeben, das auf den Überprüfungsdaten basiert.

11. Steuerprogramm nach einem der Ansprüche 9 bis 10, das weiter folgende Schritte umfasst:
• Bereitstellen (S2) von Geometriedaten, die Informationen über die Geometrie des Knochenteils (1) umfassen;
• Ermitteln (S3) einer Längsachse (5) des Knochenteils (1).

12. Steuerprogramm nach einem der Ansprüche 9 bis 11, das weiter folgenden Schritt umfasst:
Erfassen der räumlichen Lage des Werkzeugaufsatzes (26) nach dem Verbinden des Verbindungsabschnitts (24) mit der Werkzeugaufsatzführung (21), wobei die Lage in einem Koordinatensystem erfasst wird, das gleich einem Koordinatensystem ist, in dem die Lage des Knochenteils (1) beschrieben wird.

13. Steuerprogramm nach einem der Ansprüche 9 bis 12, das weiter folgenden Schritt umfasst:
Freigeben des Werkzeugaufsatzes (26), so dass keine aktiven Steuersignale mehr an ihn gesandt werden, die eine Lageveränderung des Werkzeugaufsatzes (26) bewirken.

14. Computerlesbares Medium, auf dem ein Programm gespeichert ist, das die Schritte des Steuerprogramms nach einem der Ansprüche 9 bis 13 ausführt, wenn es in eine Datenverarbeitungsvorrichtung (13) geladen wird und auf dieser abläuft.

## Claims

1. A tool attachment (26) for medical applications, in particular for fraising a part of a bone (1), in particular a femoral head, comprising:
• a connecting section (24) which is at rest relative to a part of the bone (1) while the part of the bone (1) is being machined using the tool attachment (26); and
• at least one machining section (25) for machining a part of the bone (1);
wherein
the connecting section (24) is designed to connect the tool attachment, such that it is stationary, to a tool attachment guide (21),
**characterised in that**
the tool attachment comprises a fastening facility (24') for fastening the tool attachment (26) to a control device (22), and **in that**
the machining section (25) comprises a cavity (28) which is designed to accommodate a guide marker device (23) and to enable the connecting section (24) to be connected to the tool attachment guide (21).

2. The tool attachment (26) according to claim 1, **characterised in that** a supporting section and/or the connecting section (24) comprises the cavity (28).

3. The tool attachment (26) according to claim 1, **characterised in that** the cavity (28) assists in a connection between the tool attachment (26) and the guide marker device (23).

4. The tool attachment (26) according to any one of the preceding claims, **characterised in that** the location of the cavity (28) is adjustable relative to the tool attachment (26).

5. The tool attachment (26) according to any one of claims 1 to 4, **characterised in that** the cavity (28) comprises an engaging element (27) which assists in a mechanical connection between the tool attachment (26) and the guide marker device (23).

6. A system consisting of a tool attachment (26) according to any one of the preceding claims and a guide marker device (23), wherein the connecting section (24) is designed to accommodate the guide marker device (23) in order to enable the tool attachment (26) to be connected, such that it is stationary, to the guide marker device (23).

7. A system consisting of a tool attachment (26) according to any one of claims 1 to 5 or consisting of a system according to the preceding claim and a surgical navigation system (17).

8. A control program for positioning the tool attachment (26) according to any one of claims 1 to 5 on a part of a bone (1), in particular a femoral head, by means of a control device (22), wherein said program runs on a data processing device (13) and comprises the steps of:
• providing (S4) guide data which comprise information on the location of a guide marker device (23) with respect to the part of the bone (1);
• providing location data which comprise information on the location of the tool attachment (26);
• determining control data on the basis of the guide data and the location data, wherein the control data are designed to cause the control device (22) to guide (S5) the tool attachment (26) to the guide marker device (23), such that the guide marker device (23) is accommodated in a cavity (28) provided on the tool attachment (26);
• determining (S6) connection data on the basis of the guide data, the location data and the control data, wherein said connection data comprise information on a connection between a connecting section (24) of the tool attachment (26) and the tool attachment guide (21).

9. The control program according to the preceding claim, further comprising the step of ascertaining (S8) verification data which comprise information on whether at least a part of the guide marker device (23) and/or bone marker device (9) has been removed.

10. The control program according to the preceding claim, further comprising the step of ascertaining guidance information data which cause a guidance information device to output a signal which is based on the verification data.

11. The control program according to any one of claims 9 to 10, further comprising the steps of:
• providing (S2) geometry data which comprise information on the geometry of the part of the bone (1);
• ascertaining (S3) a longitudinal axis (5) of the part of the bone (1).

12. The control program according to any one of claims 9 to 11, further comprising the step of ascertaining the spatial location of the tool attachment (26), once the connecting section (24) has been connected to the tool attachment guide (21), wherein the location is detected in a coordinate system which is identical to a co-ordinate system in which the location of the part of the bone (1) is described.

13. The control program according to any one of claims 9 to 12, further comprising the step of releasing the tool attachment (26), such that active control signals which cause a change in the location of the tool attachment (26) are no longer transmitted to the tool attachment (26).

14. A computer-readable medium on which a program is stored which performs the steps of the control program according to any one of claims 9 to 13 when it is loaded onto and run on a data processing device (13).

## Revendications

1. Accessoire d'outil (26) pour des applications médicales, en particulier pour fraiser une portion d'os (1), notamment une tête fémorale, comportant :
• une partie d'assemblage (24) qui est au repos par rapport à la portion d'os (1) pendant l'usinage d'une portion d'os (1) avec l'accessoire d'outil (26); et
• au moins une partie d'usinage (25) pour usiner une portion d'os (1);
dans lequel
la partie d'assemblage (24) est conçue pour assembler fixement l'accessoire d'outil à un guide d'accessoire d'outil (21),
**caractérisé en ce que** l'accessoire d'outil présente une possibilité de fixation (24') pour fixer l'accessoire d'outil (26) sur un dispositif de commande (22) et **en ce que**
la partie d'usinage (25) comporte une cavité (28) qui est conçue pour recevoir un dispositif de marqueur de guidage (23) et pour permettre un assemblage de la partie d'assemblage (24) avec le guide d'accessoire d'outil (21).

2. Accessoire d'outil (26) selon la revendication 1, **caractérisé en ce qu'**une partie de support et/ou la partie d'assemblage (24) comporte la cavité (28).

3. Accessoire d'outil (26) selon la revendication 1, **caractérisé en ce que** la cavité (28) contribue à un assemblage de l'accessoire d'outil (26) avec le dispositif de marqueur de guidage (23).

4. Accessoire d'outil (26) selon l'une des revendications précédentes, **caractérisé en ce que** la position de la cavité (28) peut être réglée par rapport à l'accessoire d'outil (26).

5. Accessoire d'outil (26) selon l'une des revendications 1 à 4, **caractérisé en ce que** la cavité (28) contient un élément de prise (27) qui contribue à un assemblage mécanique entre l'accessoire d'outil (26) et le dispositif de marqueur de guidage (23).

6. Système constitué d'un accessoire d'outil (26) selon l'une des revendications précédentes et d'un dispositif de marqueur de guidage (23), dans lequel la partie d'assemblage (24) est conçue pour recevoir le dispositif de marqueur de guidage (23) afin de permettre un assemblage fixe de l'accessoire d'outil (26) avec le dispositif de marqueur de guidage (23).

7. Système constitué d'un accessoire d'outil (26) selon l'une des revendications 1 à 5 ou d'un système selon la revendication précédente et d'un système de navigation chirurgical (17).

8. Programme de commande pour positionner l'accessoire d'outil (26) selon l'une des revendications 1 à 5 sur une portion d'os (1), en particulier une tête fémorale, au moyen d'un dispositif de commande (22) qui s'exécute sur un dispositif de traitement de données (13) et inclut les étapes suivantes consistant à :
• fournir (S4) des données de guidage incluant des informations sur la position d'un dispositif de marqueur de guidage (23) par rapport à la portion d'os (1);
• fournir des données de position incluant des informations sur la position de l'accessoire d'outil (26);
• déterminer des données de commande sur la base des données de guidage et des données de position, les données de commande étant conçues pour amener le dispositif de commande (22) à guider (S5) l'accessoire d'outil (26) sur le dispositif de marqueur de guidage (23) de telle sorte que le dispositif de marqueur de guidage (23) est reçu dans une cavité (28) prévue sur l'accessoire d'outil (26);
• déterminer (S6) des données d'assemblage sur la base des données de guidage, des données de position et des données de commande, incluant des informations sur un assemblage d'une partie d'assemblage (24) de l'accessoire d'outil (26) avec le guide d'accessoire d'outil (21).

9. Programme de commande selon la revendication précédente, incluant l'étape supplémentaire suivante consistant à :
déterminer (S8) des données de contrôle incluant des informations pour déterminer si au moins une partie du dispositif de marqueur de guidage (23) et/ou du dispositif de marqueur d'os (9) a été enlevée.

10. Programme de commande selon la revendication précédente, incluant en outre l'étape suivante consistant à :
déterminer des données d'information de guidage qui amènent un dispositif d'informations de guidage à délivrer un signal sur la base des données de contrôle.

11. Programme de commande selon l'une quelconque des revendications 9 à 10 incluant les étapes suivantes consistant à :
• fournir (S2) des données géométriques incluant des informations sur la géométrie de la portion d'os (1);
• déterminer (S3) un axe longitudinal (5) de la portion d'os (1).

12. Programme de commande selon l'une des revendications 9 à 11, incluant en outre l'étape suivante consistant à :
détecter la position spatiale de l'accessoire d'outil (26) après l'assemblage de la partie d'assemblage (24) avec le guide d'accessoire d'outil (21), la position étant détectée dans un système de coordonnées qui est identique à un système de coordonnées dans lequel la position de la portion d'os (1) est décrite.

13. Programme de commande selon l'une des revendications 9 à 12, incluant en outre l'étape suivante consistant à :
libérer l'accessoire d'outil (26) de telle sorte qu'aucun signal de commande actif provoquant un changement de position de l'accessoire d'outil (26), ne lui est plus envoyé.

14. Support lisible par ordinateur sur lequel est mémorisé un programme exécutant les étapes du programme de commande selon l'une des revendications 9 à 13, lorsqu'il est chargé dans un dispositif de traitement de données (13) et s'exécute sur celui-ci.
